# EUROPÄISCHE PATENTANMELDUNG

(11) **EP 3 560 922 A1**
(43) Veröffentlichungstag der Anmeldung: **30.10.2019**
(21) Anmeldenummer: 18169052.0
(22) Anmeldetag: 24.04.2018
(51) Int. Cl.: C07D 471/04

(54) **VERFAHREN ZUR HERSTELLUNG VON (4S)- 4-(4-CYANO-2-METHOXYPHENYL)-5-ETHOXY-2,8-DIMETHYL-1,4-DIHYDRO-1,6-NAPHTHYRIDIN-3-CARBOX-AMID DURCH RACEMAT-SPALTUNG MITTELS DIASTEREOMERER WEINSÄUREESTER**

(71) Anmelder: Bayer Aktiengesellschaft, 51373 Leverkusen (DE); Bayer Pharma Aktiengesellschaft, 13353 Berlin (DE)
(72) Erfinder: Die Erfindernennung liegt noch nicht vor
(74) Vertreter: BIP Patents

(57) **Zusammenfassung**

Die vorliegende Erfindung betrifft ein neues und verbessertes Verfahren zur Herstellung von (4S)- 4-(4-Cyano-2-methoxyphenyl)-5-ethoxy-2,8-dimethyl-1,4-dihydro-1,6-naphthyridin-3-carbox-amid der Formel (I) 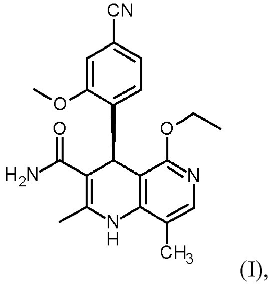 sowie die Herstellung des Enantiomeren (Ia) durch eine Racemat-Spaltung mit chiralen substituierten Weinsäureestern der allgemeinen Formeln (IIIa) und (IIIb)

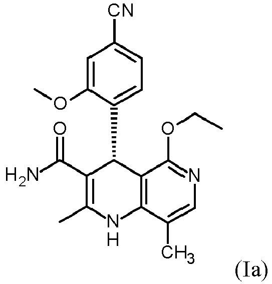

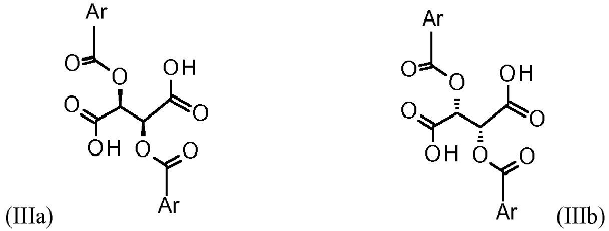

Wobei Ar für einen substituierten oder unsubstituierten Aromaten oder Heteroaromaten steht.

## Beschreibung

Die vorliegende Erfindung betrifft ein neues und verbessertes Verfahren zur Herstellung von (4S)- 4-(4-Cyano-2-methoxyphenyl)-5-ethoxy-2,8-dimethyl-1,4-dihydro-1,6-naphthyridin-3-carbox-amid der Formel (I) 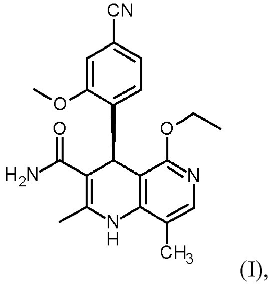 sowie die Herstellung des Enantiomeren (Ia) durch eine Racemat-Spaltung mit chiralen substituierten Weinsäureestern der allgemeinen Formeln (IIIa) und (IIIb)

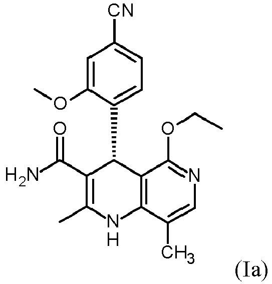

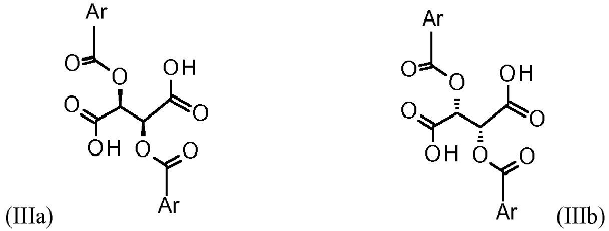

Wobei Ar für einen substituierten oder unsubstituierten Aromaten oder Heteroaromaten steht.

Finerenone (I) wirkt als nicht steroidaler Antagonist des Mineralcorticoid Rezeptors und kann als Mittel zur Prophylaxe und /oder Behandlung von kardiovaskulären und renalen Erkrankungen wie beispielsweise Herzinsuffizienzen und diabetische Nephropathie eingesetzt werden.

Die Verbindung der Formel (I) und deren Herstellungsprozess sind in der WO 2008/104306 und ChemMedChem 2012, 7, 1385 sowie in WO 2016/016287 A1 beschrieben. Um an die Verbindung der Formel (I) zu gelangen, muss das racemische Gemisch der Amide (II) 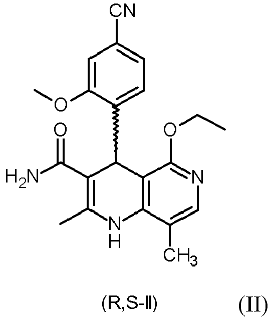 in die Antipoden getrennt werden, da nur der Antipode der Formel (I) aktiv ist.

In der publizierten Forschungs-Synthese (WO 2008/104306) wurde hierzu eine speziell synthetisierte chirale Phase verwendet (Eigenherstellung), die als chiralen Selektor Poly(*N*-methacryloyl-D-leucin-dicyclopropylmethylamid enthielt. Es wurde gefunden, dass man die Trennung auch auf einer kommerziell leicht zugänglichen Phase vornehmen kann. Hierbei handelt es sich um die Phase Chiralpak AS-V, 20 µm. Als Laufmittel wurde eine Mischung aus Methanol/Acetonitril 60:40 verwendet. Hierbei kann die Chromatographie an einer handelsüblichen Chromatographie-Säule durchgeführt werden, bevorzugt werden aber mit dem Fachmann bekannten Techniken wie SMB (simulated moving bed; G. Paredes, M. Mazotti, Journal of Chromatography A, 1142 (2007): 56-68) oder Varicol (Computers and Chemical Engineering 27 (2003) 1883-1901) eingesetzt.

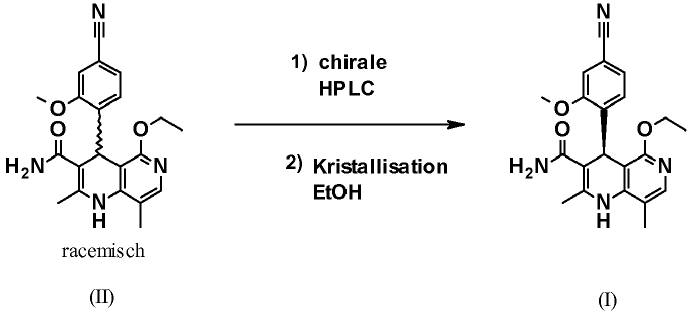

Obwohl die SMB-Trennung eine recht gute Ausbeute und optische Reinheit liefert, sind die Anschaffungskosten und Betreibung einer solchen Anlage unter GMP-Bedingungen eine hohe Herausforderung und mit hohen Kosten verbunden. Auch die jeweils verwendete chirale Phase ist sehr teurer und hat nur eine begrenzte Lebenszeit und muss in einer laufenden Produktion immer wieder ausgewechselt werden. Dieses ist aus produktionstechnischen Gründen nicht optimal, wenn nicht eine zweite Anlage vorhanden ist, damit ein Dauerbetrieb gewährleistet ist, was mit noch zusätzlichen Kosten verbunden ist. Des Weiteren ist vor allem bei Produkten, die im Tonnenmaßstab gefertigt werden die Rückgewinnung des Lösungsmittels der zeitbestimmende Schritt und erfordert die Anschaffung riesiger Fallfilm-Verdampfer und ist mit dem Verbrauch enormer Energiemengen verbunden.

Es bestand daher die Aufgabe nach Alternativen zur Auftrennung des Enantiomeren-Gemisches zu suchen, die signifikant kostengünstiger sind und mit herkömmlichem Pilot-Plant-Equipment (Rührkessel/Isolierapparate) durchzuführen sind. Derartige Anlagen gehören traditionell zu der Standard-Ausrüstung von pharmazeutischen Produktionsbetrieben und erfordern keine zusätzlichen Investitionen. Auch ist die Qualifizierung und Validierung von Batch-Prozessen um vieles einfacher als bei chromatographischen Verfahren, was von zusätzlichem Vorteil ist.

Es wurden zahlreiche Versuche unternommen, mit den üblichen klassischen Methoden zur Racemat-Spaltung eine Trennung auszuarbeiten (Variation von chiraler organischer Säure und Lösungsmittel), wie in Tabelle 1 dargestellt:

**Tabelle1:**

| **Säure** | **Lösungsmittel** |
|---|---|
| (S)-(+)-1, 1 -Binaphtyl-2,2-diylhydrogenphosphat | Methanol |
| (-)-Chinasäure | Ethanol |
| (-)-O,O'-Diacetyl-L-weinsäure | 1-Propanol |
| (-)-O,O'-Dipivaloyl-L-weinsäure | 2-Propanol |
| (+)-3-Bromcampher-10-sulfonsäure | 1-Butanol |
| (+)-4-Chloro-tartranilic acid | Isobutanol |
| (+)-4'-Nitrotartranilic acid | 1-Pentanol |
| (+)-Camphersäure | Cyclohexanol |
| (+)-O-Acetyl- L-mandelsäure | Benzylalkohol |
| (1 R)-(-)-Campher-1 0-sulfonsaeure | Aceton |
| (1S)-(-)-Camphansäure | Ethylenglycol |
| **(2R,3R)-(+)-Weinsäure** | Chlorbenzol |
| (R)-(+)-alpha-Methoxy-alpha-trifluoromethylphenylacetic acid | Dichlormethan |
| (S)-(-)-2-Bromopropionic acid | Ethylacetat |
| (S)-(-)-2-Chloropropionic acid | Dimethylacetamid |
| (S)-(+)-Citramalsäure | THF |
| (S)-(+)-Mandelsäure | Toluol |
| Ac-Acid-Triple-Mix | Toluol / Ethylacetat 95:5 |
| Äpfelsäure (Malicacid) | Toluol / Ethylacetat 90:10 |
| **Chlocyphos** | Toluol / Ethylacetat 80:20 |
| D-(+)-HPP-Mono-Säure | Toluol / Ethyacetat 50:50 |
| L-Glutaminsäure | Toluol / Ethanol 95:5 |
| L-Milchsäure | Methanol / Wasser 90:10 |
| Menthoxyessigsäure | Methanol / Wasser 80:20 |
| N-(3,5-Dinitrobenzoyl)-(R)-(-)-2-phenylglycin | Methanol / Wasser 50:50 |
| N-Acetyl-L-leucine | Ethylacetat / MeOH 90:10 |
| N-Acetyl-L-Phenylalanine | Ethanol / Wasser 90:10 |
| N-Ac-Prolin-OH | Ethanol / Wasser 85:15 |
| Naproxen | Ethanol / Wasser 80:20 |
| Phencyphos | Ethanol / Wasser 75:25 |
| | Ethanol / Wasser 70:30 |
| | Ethanol / Wasser 50:50 |
| | Methanol / Wasser 90:10 |
| | Methanol / Wasser 80:20 |
| | Methanol / Wasser 50:50 |
| | 1-Propanol / Wasser 80:20 |
| | Isopropanol / Wasser 80:20 |
| | 1-Butanol / Wasser 90:10 |
| | 2-Butanol / Wasser 80:20 |
| | 2-Butanol / Wasser 90:10 |
| | 1-Pentanol / Wasser 90:10 |
| | Cyclohexanol / Wasser 90:10 |
| | Benzylalkohol / Wasser 90:10 |
| | Ethylenglycol / Wasser 80:20 |

Unter anderem wurden auch Experimente mit dem klassischen Spaltungs-Reagenz (+)- Weinsäure durchgeführt.

Jedoch wurde in allen Fällen keine Salzbildung beobachtet, stattdessen fällt immer nur das Racemat, unversalzt aus der Lösung aus. Dieses entspricht im Wesentlichen den Erwartungen des Fachmanns, die man aus dem pKs-Wert des Moleküls (II) hätte ableiten können, dass eine klassische Racemat-Spaltung mittels Diastereomerer Salzbildung mit organischen Säuren nicht möglich sein sollte, da der gemessene pKs-Wert (für die Base) bei 4,3 liegt und eine Versalzung damit nahezu ausschließt, sodass nur eine Salzbildung unter Verwendung sehr starker anorganischer oder organischer Mineral-Säuren, wie z.B. chiraler Sulfonsäuren oder Phosphonsäuren bevorzugt möglich sein sollte. Laut Literatur, wie z.B. "Handbook of Pharmaceutical Salts - Properties, Selection and Use; von P. Heinrich Stahl, Camille G. Wermuth (Eds.); Wiley-VCH, S.166" sollte der pK-Wert-Unterschied mindestens 3 pK-Einheiten betragen, um eine stabile Salzbildung zu ermöglichen. Dieses wird in der Tat gefunden, wenn man beispielsweise nachfolgend aufgeführten cyclischen Phosphorsäureester Chlocyphos einsetzt:

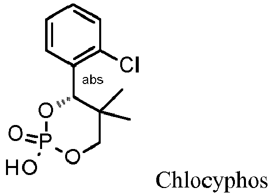

Bei der Umsetzung mit 3 eq. dieses cyclischen Phosphorsäureesters mit dem Racemat (II) erhält man ein Diastereomeren-Salz worin (I) mit einem Enantiomeren-Überschuss von lediglich 44% e.e. vorliegt.

Alle Bemühungen den Enantiomeren-Überschuss in Richtung > 99% e.e. zu bringen waren nicht zielführend; zudem war Chlocyphos nicht in größeren Mengen kommerziell erhältlich, daher wurde nach weiteren Alternativen gesucht.

Bei der Umsetzung mit Alkyl-substituierten Weinsäurederivaten wie beispielsweise (-)-O,O'-Dipivaloyl-L-weinsäure oder (-)-O,O'-Diacetyl-L-weinsäure konnte keine Salzbildung beobachtet werden.

Bei der weiteren Bearbeitung des Themas wurde überraschenderweise gefunden, dass aromatisch bzw heteroaromatisch substituierte Derivate der Weinsäure ausgezeichnet geeignet sind, um "Diastereomerensalze" aus dem Racemat (II) zu bilden.

Gegenstand der vorliegenden Anmeldung ist daher die Racemat-Spaltung von (II) 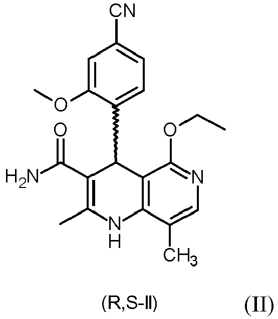 in (Ia) und/oder (I) 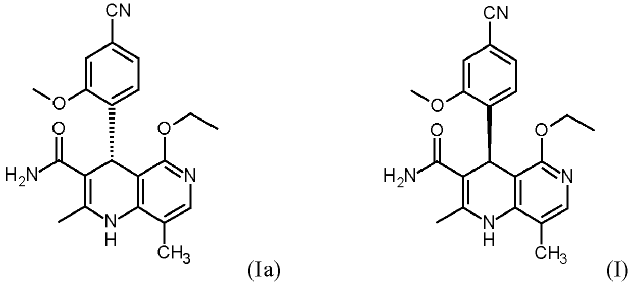 mit chiralen substituierten Weinsäureestern der allgemeinen Formeln (IIIa) oder (IIIb) 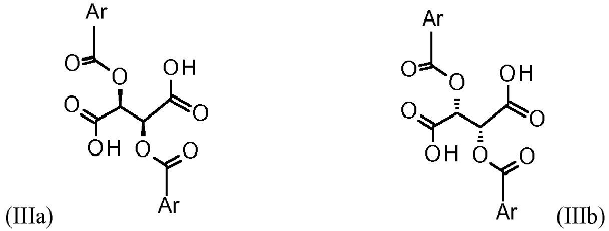 wobei Ar für einen unsubstituierten oder substituierten Aromaten oder Heteroaromaten steht.

Ein weiterer Gegenstand betrifft Verfahren zur Herstellung von (4S)- 4-(4-Cyano-2-methoxyphenyl)-5-ethoxy-2,8-dimethyl-1,4-dihydro-1,6-naphthyridin-3-carbox-amid der Formel (I) 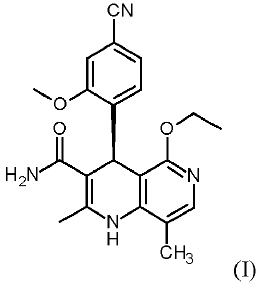 durch Racematspaltung von (II) 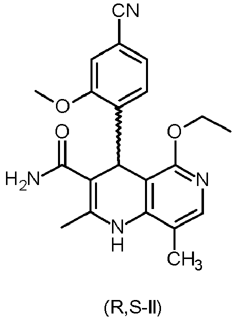 mit einem chiralen substituierten Weinsäureester der Formel (IIIa) 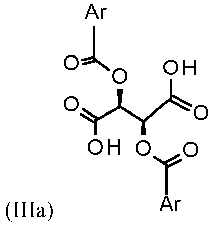 wobei Ar für einen unsubstituierten oder substituierten Aromaten oder Heteroaromaten steht.

Der Begriff "substituiert" bedeutet, dass ein oder mehrere Wasserstoffatome an dem betreffenden Atom bzw. der betreffenden Gruppe durch eine Auswahl aus der angegebenen Gruppe ersetzt ist/sind, mit der Maßgabe, dass die normale Valenz des betreffenden Atoms unter den vorliegenden Umständen nicht überschritten wird. Kombinationen von Substituenten und/oder Variablen sind zulässig.

Der Begriff "unsubstituiert" bedeutet, dass kein Wasserstoffatom ersetzt wurde.

Bei der Heteroaryl-Gruppe kann es sich um eine 5-gliedrige Heteroaryl-Gruppe wie beispielsweise Thienyl, Furanyl, Pyrrolyl, Oxazolyl, Thiazolyl, Imidazolyl, Pyrazolyl, Isoxazolyl, Isothiazolyl, Oxadiazolyl, Triazolyl, Thiadiazolyl oder Tetrazolyl; oder um eine 6-gliedrige Heteroaryl-Gruppe wie beispielsweise Pyridinyl, Pyridazinyl, Pyrimidinyl, Pyrazinyl oder Triazinyl; oder um eine tricyclische Heteroarylgruppe wie beispielsweise Carbazolyl, Acridinyl oder Phenazinyl; oder um eine 9-gliedrige Heteroarylgruppe wie beispielsweise Benzofuranyl, Benzothienyl, Benzoxazolyl, Benzisoxazolyl, Benzimidazolyl, Benzothiazolyl, Benzotriazolyl, Indazolyl, Indolyl, Isoindolyl, Indolizinyl oder Purinyl; oder um eine 10-gliedrige Heteroaryl-Gruppe wie beispielsweise Chinolinyl, Chinazolinyl, Isochinolinyl, Cinnolinyl, Phthalazinyl, Chinoxalinyl oder Pteridinyl handeln.

Insbesondere handelt es sich bei der Heteroaryl-Gruppe um eine Pyridinyl, Pyrazinyl, Pyrrolyl, Pyrazolyl oder Pyrimidinyl-Gruppe.

Aryl-Gruppe im Sinne der vorliegenden Anmeldung ist insbesondere eine Phenylgruppe.

Als Substituenten im Sinne der vorliegenden Erfindung kommen Halogen, C₁-C₆-Alkyl, C₁-C₆-Alkoxy, Nitril, eine Nitrogruppe, Cyanogruppe, CF3-Gruppe oder Amidgruppe, wie zum Beispiel NHCOR, in der R für Methyl, Ethyl oder Phenyl stehen kann, oder eine -NRCOR-Gruppe in der R die oben genannte Bedeutung hat oder eine CONHR-in der R die oben genannte Bedeutung hat, oder eine CONRR', in der R'gleichbedeutend mit R ist wie oben definiert oder cyclische Amide wie - COMorpholin-Rest, -COPiperidin-Rest

Der Begriff Halogen bezeichnet ein Fluor-, Chlor-, Brom- oder Iodatom, insbesondere ein Fluor-, Chlor- oder Bromatom.

Der Begriff "C₁-C₆-Alkyl" bedeutet eine geradkettige oder verzweigte gesättigte monovalente Kohlenwasserstoffgruppe mit 1, 2, 3, 4, 5 oder 6 Kohlenstoffatomen, z. B. eine Methyl-, Ethyl-, Propyl-, Isopropyl-, Butyl-, *sec*-Butyl-, Isobutyl-, *tert*-Butyl-, Pentyl-, Isopentyl-, 2-Methylbutyl-, 1-Methylbutyl-, 1-Ethylpropyl-, 1,2-Dimethylpropyl-, Neopentyl-, 1,1-Dimethylpropyl-, Hexyl-, 1-Methylpentyl-, 2-Methylpentyl-, 3-Methylpentyl-, 4-Methylpentyl-, 1-Ethylbutyl-, 2-Ethylbutyl-, 1,1-Dimethylbutyl-, 2,2-Dimethylbutyl-, 3,3-Dimethylbutyl-, 2,3-Dimethylbutyl-, 1,2-Dimethylbutyl- oder 1,3-Dimethylbutylgruppe oder ein Isomer davon. Die Gruppe weist insbesondere 1, 2, 3 oder 4 Kohlenstoffatome auf ("C₁-C₄-Alkyl"), z. B. eine Methyl-, Ethyl-, Propyl-, Isopropyl-, Butyl-, *sec-*Butyl-, Isobutyl- oder *tert*-Butylgruppe, insbesondere 1, 2 oder 3 Kohlenstoffatome ("C₁-C₃-Alkyl"), z. B. eine Methyl-, Ethyl-, *n*-Propyl- oder Isopropylgruppe.

Der Begriff "C₁-C₆-Alkoxy" bedeutet eine geradkettige oder verzweigte gesättigte monovalente Gruppe der Formel (C₁-C₆-Alkyl)-O-, in welcher der Begriff "C₁-C₆-Alkyl" wie oben definiert ist, z. B. eine Methoxy-, Ethoxy-, *n*-Propoxy-, Isopropoxy-, *n*-Butoxy-, *sec*-Butoxy-, Isobutoxy-, *tert*-Butoxy-, Pentyloxy-, Isopentyloxy- oder *n*-Hexyloxygruppe oder ein Isomer davon.

Bevorzugt steht Ar für:

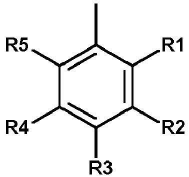

Wobei R1, R2, R3, R4, R5 jeweils für ein Wasserstoffatom oder einen Alkylrest, wie zum Beispiel Methyl, Ethyl, Propyl oder ein Halogenatom, wie zum Beispiel Fluor, Chlor, Brom oder Jod oder eine Ethergruppe, wie zum Beispiel O-Methyl, O-Ethyl, O-Phenyl, oder eine Nitrogruppe, Cyanogruppe, CF3-Gruppe oder Amidgruppe, wie zum Beispiel NHCOR, in der R für Methyl, Ethyl oder Phenyl stehen kann, oder eine -NRCOR-Gruppe in der R die oben genannte Bedeutung hat oder eine CONHR-in der R die oben genannte Bedeutung hat, oder eine CONRR', in der R'gleichbedeutend mit R ist wie oben definiert oder cyclische Amide wie -COMorpholin-Rest, -COPiperidin-Reststeht. Die Substitutionsmuster können sehr unterschiedlich sein, so können theoretisch bis zu 5 verschiedene Substituenten möglich sein, in der Regel sind aber die monosubstituierten Ar-Reste bevorzugt. Ar kann aber auch ein substituierter Heteroaromat, wie vorzugsweise. Pyridin oder Pyrazin sein. Es kann sich auch um einen policyclischen aromatischen Kohlenwasserstoff, wie. z.B. ein substituiertes Naphthalin, Anthrazen, oder Chinolin handeln.

Besonders bevorzugt als Ar sind: 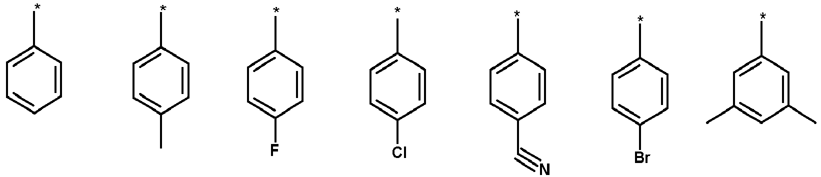 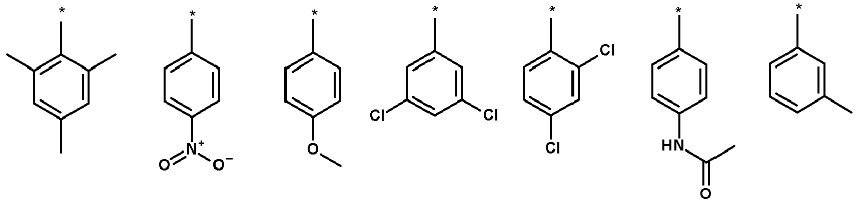 worin * für die Verknüpfungsstelle steht.

Insbesondere bevorzugt steht Ar für: 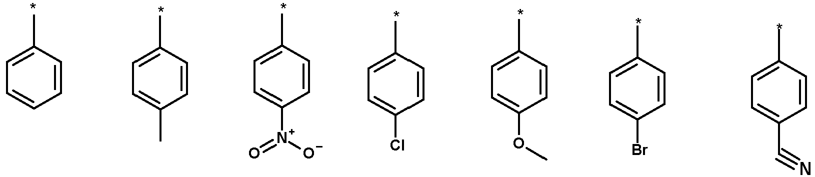 worin * für die Verknüpfungsstelle steht.

Ganz besonders bevorzugte Ar-Reste sind: 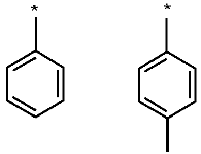 worin * für die Verknüpfungsstelle steht.

Von denen der unsubstituierte Ring (Phenyl) in besonderer Weise bevorzugt ist.

Die Herstellung der Weinsäureester ist literaturbekannt, wie z.B. in Organic Synthesis, Coll. Vol. 9, p.722 (1998); Vol. 72, p.86 (1995), sowie in Chirality 2011 (23), 3, p.228 beschrieben.

Ein ebenfalls weiterer Gegenstand betrifft Diastereomerensalze gemäß der Formeln 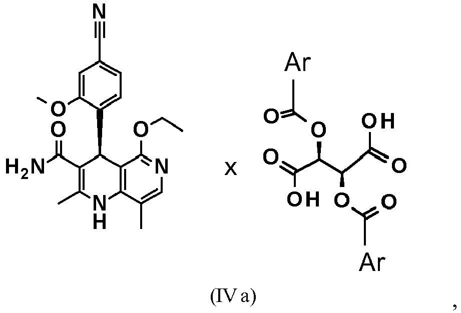 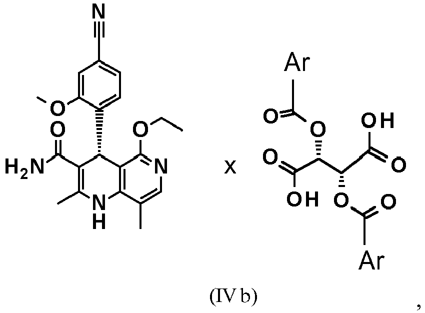 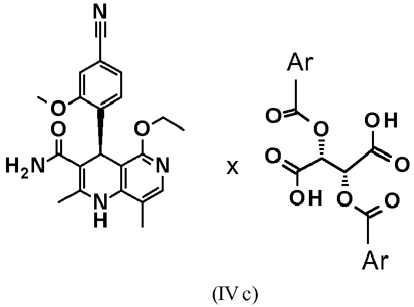 oder 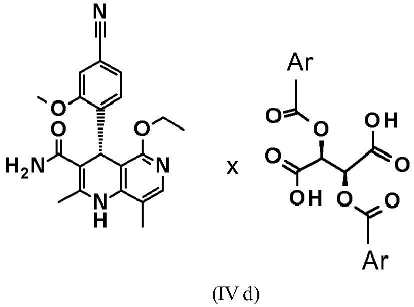 worin Ar für einen unsubstituierten oder substituierten Aromaten oder Heteroaromaten steht und die oben angegebene Bedeutung hat.

Besonders bevorzugt sind Diastereomerensalze in denen Ar für Phenyl steht.

Ob es sich dabei wirklich um ein klassisches Diastereomeren-Salz oder ein über Wasserstoff-Brückenbindung stabilisierte 1:1 Molekül-Komplex handelt ist nicht eindeutig vorhersagbar. Fest steht aber, dass diese 1:1 Molekül-Aggregate sehr stabil sind und sich wie klassische Diastereomeren-Salze verhalten und isolieren lassen, sodass wir im Nachfolgenden doch den Namen Diastereomeren-Salz verwenden. Für die Darstellung der Diastereomerensalze werden Weinsäure-Derivate der allgemeinen Formel (IIIa) und (IIIb) verwendet: 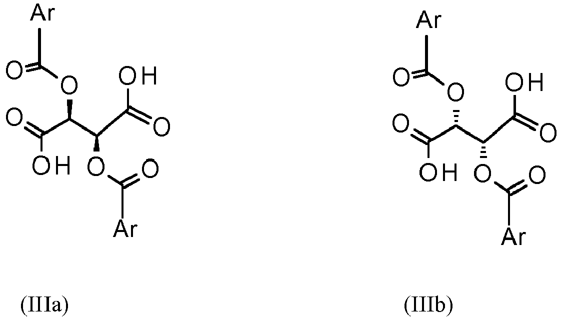 worin Ar für einen substituierten oder unsubstituierten Aromaten oder Heteroaromaten steht.

Die Herstellung der Diastereomeren-Salze wird wie folgt durchgeführt:

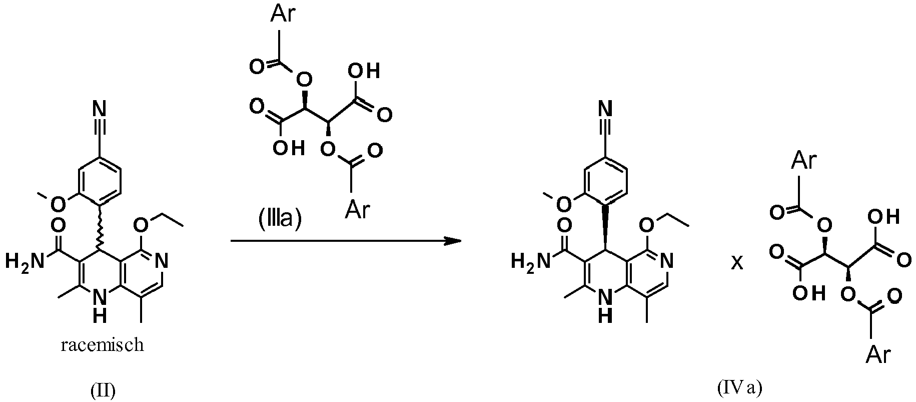

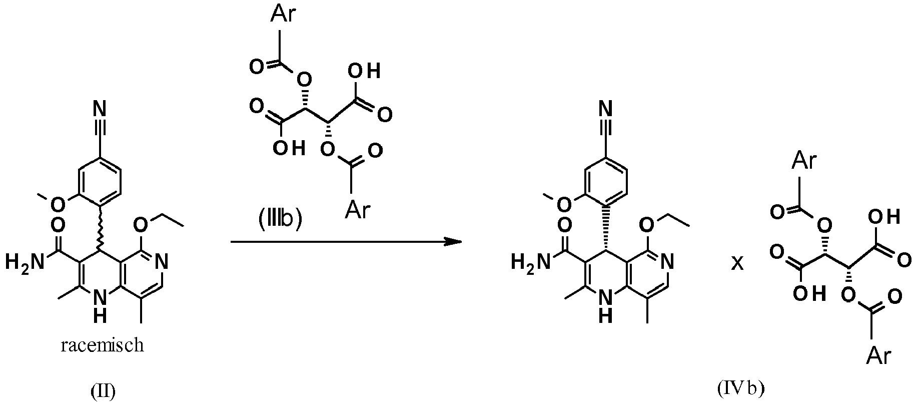

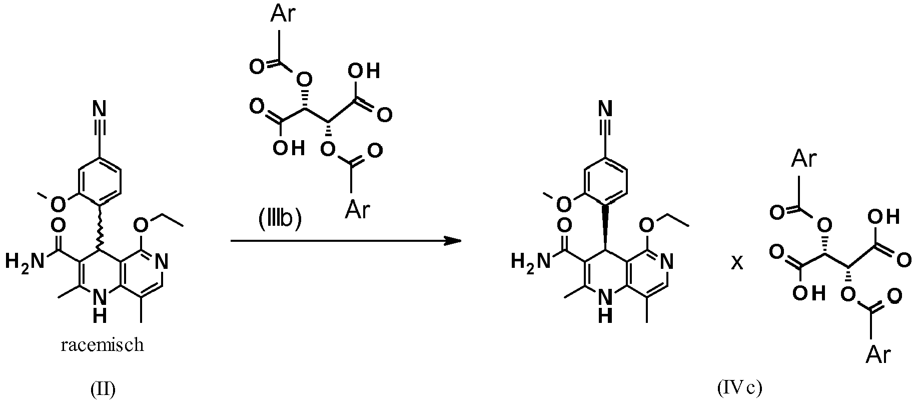

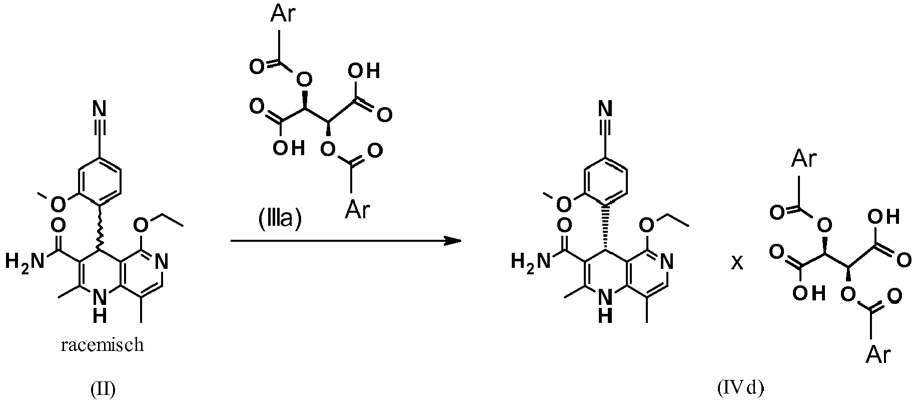

Die Umsetzung des racemischen Gemisches (II) mit einem Weinsäure-Derivat der allgemeinen Formel (IIIa) oder (IIIb) ergibt die 4 Möglichkeiten der Diastereomeren-Salz-Bildung (IV a-d). Es wird überaschenderweise eine gewisse Präferenz dahingehend beobachtet, dass wenn man zum Beispiel rac-(II) mit einem Weinsäure-Derivat der allgemeinen Formel (IIIa) umsetzt man in den meisten Fällen das Diastereomeren-Salz der allgemeinen Formel (IVa) erhält, wobei das Antipode der S-Konfiguration bevorzugt die Salzbildung eingeht. Das Diastereomeren-Salz (IVa) fällt nahezu quantitativ aus der Lösung aus, aus der es anschließend z.B. durch Filtration isoliert werden kann, wobei das Antipode mit der R-Konfiguration in Lösung bleibt. In ganz ähnlich überaschenderweise wird das spiegelbildliche Salz der allgemeinen Form (IVb) hergestellt, indem man das Racemat (II) mit dem Weinsäure-Derivat der allgemeinen Formel (IIIb) umsetzt, wobei das Antipode der R-Konfiguration bevorzugt die Salzbildung eingeht. Die ausgefallenen Diastereomeren-Salze können nahezu quantitativ abgetrennt werden, wobei hier der S-Antipode in Lösung verbleibt.

Durch das stöchiometrische Verhältnis von (II) zu (IIIa) respektive (IIIb) sowie durch die Auswahl des Lösungsmittels lässt sich die Ausbeute und die Enantiomerenreinheit optimieren.

Da Finerenone (I) die S-Konfiguration besitzt, werden bevorzugt die S,S konfigurierten Weinsäure-Derivate zur Racemat-Spaltung eingesetzt, da in diesem Falle bevorzugt das Diastereomeren-Salz des S-Antipoden gebildet wird.

Es werden 0,4 bis 1,2 eq. Weinsäureester (IIIa) oder (IIIb) für die Racemat-Spaltung eingesetzt, bevorzugt jedoch 0,4 bis 0,7 eq. besonders bevorzugt jedoch 0,45 bis 0,6 eq., ganz besonders bevorzugt aber 0,50 -0,55 eq.

Die Diastereomeren-Salzbildung erfolgt in Lösungsmittelgemischen, die aus Wasser und mit Wasser mischbaren organischen Lösungsmitteln bestehen.

Als organische Lösungsmittel im Sinne der Anmeldung eignen sich beispielsweise Ethanol, Methanol, Isopropanol, 1-Propanol, Ethylacetat, Isobutanol, Dichlormethan, 1-Pentanol oder Aceton, bevorzugt wird aber Ethanol eingesetzt. Die Lösungsmittel können auch in der handelsüblichen vergällten Form, wie die beim Ethanol verwendeten Vergällungsmittel z.B. Toluol, Methylethylketon, Thiophen, Hexan eingesetzt werden, was aus kostentechnischen Gründen große Vorteile mit sich bringt daher eignet sich insbesondere für den großtechnischen Einsatz Branntwein welcher im Sinne der Anmeldung aus Ethanol das gegebenenfalls mit Toluol oder Methylethylketon vergällt sein kann besteht. Im Sinne der vorliegenden Anmeldung soll daher wenn im folgenden Ethanol als Lösungsmittel genannt ist darunter neben reinem Ethanol auch, insbesondere für den grosstechnischen Einsatz, Branntwein im Sinne der oben genannten Definition verstanden werden. Darüber hinaus wurden auch noch nachfolgende Lösungsmittel verwendet: Ethylacetat / Methanol 90:10; Methanol / Wasser 80:20; Ethanol / Wasser 90:10; Ethanol / Wasser 85:15; Ethanol / Wasser 80:20; Ethanol / Wasser 75:25; Ethanol / Wasser 70:30; Dichlormethan; 1-Propanol / Wasser 80:20; 1-Pentanol; 1-Pentanol / Wasser 90:10; Isopropanol; Isopropanol / Wasser 80:20; Isobutanol / Wasser 90:10; Isobutanol / Wasser 80:20; Cyclohexanol / Wasser 90:10; Benzylalkohol / Wasser 90:10; Ethylenglycol; Ethylenglycol / Wasser 80:20.

Bevorzugt wird die Racematspaltung in Ethanol/ Wasser Gemisch durchgeführt,wobei Mischungen (Vol/Vol) im Bereich von Ethanol: Wasser = 1: 1 bis 6:1 liegt. Bevorzugt wird aber eine Mischung von Ethanol: Wasser = 4: 1 bis 3: 1 verwendet. Besonders bevorzugt wird eine Mischung von Ethanol: Wasser = 3: 1 verwendet. Die Mischung kann zuvor hergestellt werden, oder aber in situ, nach Vorlage aller Komponenten in einem Topf erzeugt werden. Das Lösungsmittelgemisch kann in 10 bis 40fachem Überschuss bezogen auf das Racemat (II) eingesetzt werden, e.g. auf 1kg Racemat werden 10L bis 40L Lösungsmittelgemisch eingesetzt. Bevorzugt ist ein 10 bis 20facher Überschuss, insbesondere 13 bis 16facher Überschuss - besonders bevorzugt ist ein 14 bis 15 facher Überschuss.

Üblicherweise erfolgt die Racematspaltung indem man zuerst alle Komponenten in dem Lösungsmittelgemisch bei Raumtemperatur vorlegt, anschließend auf 60 bis 80°C, bevorzugt aber auf 75 °C erwärmt und 2 bis 10 Stunden, bevorzugt 3 bis 4 Stunden bei 75°C nachrührt und anschließend innerhalb von 3 bis 10 Stunden, bevorzugt 4 bis 5 Stunden auf Raumtemperatur (ca. 20 bis 23°C) abkühlt (mittels einer Temperatur-Rampe). Anschließend lässt man 2 bis 24 Stunden, bevorzugt 5 bis 18 Stunden, ganz bevorzugt 12 bis 16 Stunden bei Raumtemperatur nachrühren. Die Racematspaltung erfolgt bevozugt bei einer Temperatur von 75°C

Im Anschluss isoliert man das ausgefallene Diastereomerensalz (IVa), (IVb), (IVc) und/ oder (IVd).

Die Isolierung erfolgt nach dem Fachmann bekannten Methoden, wie zum Beispiel durch Filtration oder über eine Zentrifuge. Der so erhaltene Filterkuchen kann einmal, oder mehrmals mit einem Lösungsmittel oder Lösungsmittel Gemisch nachgewaschen werden. Anschließend erfolgt eine Trocknung unter Vakuum, bevorzugt < 100 mbar bei erhöhter Temperatur (50 bis 80°C, bevorzugt 50°C). Die Verwendung von Schleppgas hat sich in manchen Fällen als vorteilhaft erwiesen.

Mit der oben geschilderten Verfahrensweise gelingt es chemisch sehr reine Diastereomeren-Salze herzustellen. Der Enantiomeren-Überschuss der Diastereomeren-Salze liegt in der Regel > 97 % e.e. (siehe Beispiele)

Die Diastereomerensalze müssen nicht unbedingt getrocknet werden, sondern können auch feucht in der nächsten Prozessstufe eingesetzt werden.

Die Verfahrensschritte können neben der üblichen oben genannten Vorgehensweise auch kombiniert oder in der Reihenfolge getauscht werden, wie in der folgenden Tabelle 2 dargestellt:

**Tabelle 2**

| **1. Schritt** | **2. Schritt** | **3. Schritt** | **4. Schritt** | **5. Schritt** |
|---|---|---|---|---|
| Vorlegen vom Racemat (II) und Diaryl- Weinsäure (IIIa oder b) | Zugabe von Ethanol | Zugabe von Wasser | Erwärmen | |
| Vorlegen von Racemat (II) | Zugabe von Ethanol | Zugabe von Diaryl-Weinsäure (IIIa oder b) | Zugabe von Wasser | Erwärmen |
| Vorlegen von Racemat (II) | Zugabe von Wasser | Zugabe von Diaryl-Weinsäure (IIIa oder b) | Zugabe von Ethanol | Erwärmen |
| Vorlegen von Diaryl-Weinsäure (IIIa oder b) | Zugabe von Ethanol | Zugabe von Racemat (II) | Zugabe von Wasser | Erwärmen |
| Vorlegen von Diaryl-Weinsäure (IIIa oder b) | Zugabe von Wasser | Zugabe von Racemat (II) | Zugabe von Ethanol | Erwärmen |
| Vorlegen vom Racemat (II) und Diaryl-Weinsäure (IIIa oder b) | Zugabe des Ethanol-Wasser Gemisches | Erwärmen | | |
| Vorlegen von Racemat (II) | Zugabe des Ethanol-Wasser Gemisches | Zugabe von Diaryl-Weinsäure (IIIa oder b) | Zugabe von Ethanol | Erwärmen |
| Vorlegen von Diaryl-Weinsäure (IIIa oder b) | Zugabe des Ethanol-Wasser Gemisches | Zugabe von Racemat (II) | Zugabe von Wasser | Erwärmen |
| Ethanol-Wassergemisch vorlegen | Zugabe von Racemat (II) | Zugabe von Diaryl-Weinsäure (IIIa oder b) | Erwärmen | |
| Ethanol-Wassergemisch vorlegen | Zugabe von Diaryl-Weinsäure (IIIa oder b) | Zugabe von Racemat (II) | Erwärmen | |
| Vorlegen von Ethanol | Zugabe von Racemat (II) | Zugabe von Wasser | Zugabe von Diaryl-Weinsäure (IIIa oder b) | Erwärmen |
| Vorlegen von Wasser | Zugabe von Racemat (II) | Zugabe von Ethanol | Zugabe von Diaryl-Weinsäure (IIIa oder b) | Erwärmen |
| Vorlegen von Ethanol | Zugabe von Diaryl-Weinsäure (IIIa oder b) | Zugabe von Wasser | Zugabe von Racemat (II) | Erwärmen |
| Vorlegen von Wasser | Zugabe von Diaryl-Weinsäure (IIIa oder b) | Zugabe von Ethanol | Zugabe von Racemat (II) | Erwärmen |

Je nach Anlagen-Typ in der Pilot-Anlage, bzw. in der Produktion kann die eine oder andere Variante von Vorteil sein.

Im nächsten Schritt behandelt man das Diastereomerensalz mit einer Base und entfernt das Lösungsmittel. Die Entfernung des Lösungsmittels erfolgt nach dem Fachmann bekannten Methoden, beispielsweise durch abdestillieren. Zur Herstellung der chiralen Verbindungen (I) und (Ia) muss das Diastereomerensalz der allgemeinen Formel (IVa), (IVb), (IVc) oder (IVd) mit einer Base behandelt werden, dabei fällt das Ziel-Molekül (I) oder (Ia) nach abdestillieren des organischen Lösungsmittels aus der Lösung aus, wird isoliert - beispielsweise durch abfiltrieren und nachgewaschen und der jeweilige Weinsäureester gemäß Formel (IIIa) oder (IIIb) verbleibt in versalzter Form in Lösung.

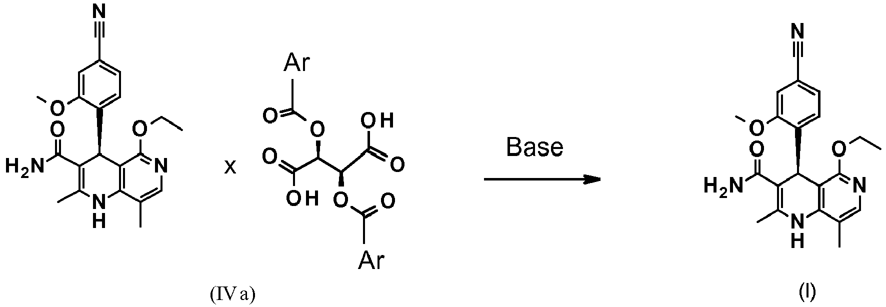

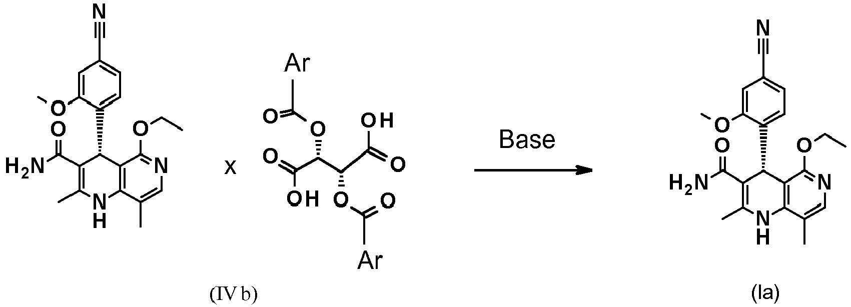

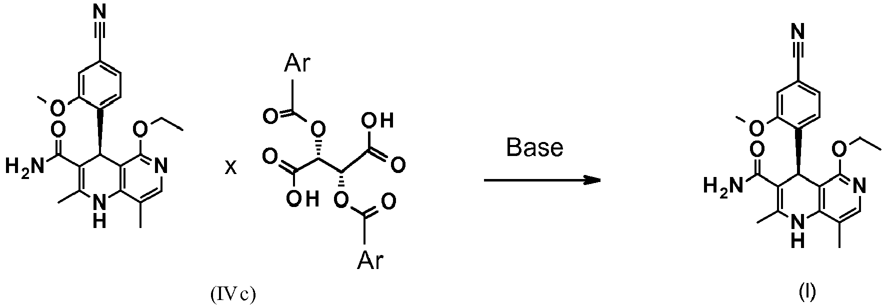

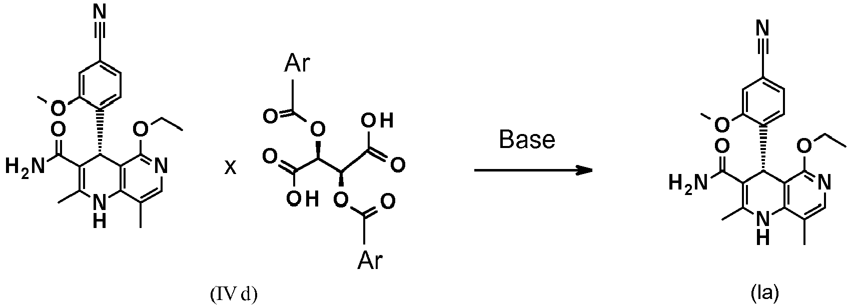

Als Basen im Sinne der vorliegenden Erfindung eignen sich anorganische und organische Basen. Im Falle von anorganischen Basen können Ammoniak, Natronlauge, Lithiumhydroxid, Kaliumhydroxid, Ammoniumcarbonat, Natriumcarbonat, Kaliumcarbonat, Lithiumcarbonat, Ammoniumhydrogencarbonat, Natriumhydrogencarbonat, Kaliumhydrogencarbonat, Natriumphosphat, Kaliumphosphat, Ammoniumphosphat eingesetzt. Bevorzugt wird jedoch Natriumhydroxid, Natriumphosphat oder Kaliumphosphat verwendet. Besonders bevorzugt wird Natriumphosphat oder Kaliumphosphat verwendet. Es ist wichtig zu betonen, dass die anorganischen Basen sowohl in wasserfreier Form, als auch in Form ihrer Hydrate eingesetzt werden können, so kann z.B. Natriumphosphat (wasserfrei) und Natriumphosphat-Hydrat erfolgreich verwendet werden. Als organische Base können aliphatische oder aromatische Basen, wie z.B. Triethylamin, Imidazol, N-Methylimidazol, Hünigbase, Pyridin, 1,8-Diazabicyclo[5.4.0]undec-7-en (DBU) verwendet werden.

Die Freisetzung der Zielverbindung (I) oder (Ia) erfolgt in Gemischen von Wasser, mit Wasser mischbaren organischen Lösungsmitteln, wie Ethanol, Isopropanol, 1,2-Ethandiol, Methoxyethanol, Methanol oder Aceton, bevorzugt wird aber Ethanol eingesetzt. Die Lösungsmittel können auch in der handelsüblichen vergällten Form, wie die beim Ethanol verwendeten Vergällungsmittel z.B. Toluol, Methylethylketon, Thiophen, Hexan eingesetzt werden, bevorzugt wird Branntwein welcher im Sinne der Anmeldung aus Ethanol das gegebenenfalls mit Toluol oder Methylethylketon vergällt sein kann besteht verwendet, was aus kostentechnischen Gründen große Vorteile mit sich bringt. Es hat sich als vorteilhaft erwiesen Mischungen von Wasser und Ethanol zu verwenden, wobei Mischungen (Vol/Vol) im Bereich von Ethanol: Wasser = 1: 6 bis 1: 3 liegt. Bevorzugt wird aber eine Mischung von Ethanol: Wasser = 1: 4 verwendet. Die Mischung kann zuvor hergestellt werden, oder aber in situ, nach Vorlage aller Komponenten in einem Topf erzeugt werden. Es können 7 bis 20 fach diese Gemisches bezogen auf eingesetztes Diastereomerensalz (IVa oder IVb oder IVc oder IVd) verwendet werden, also z.B. 1 kg in 7 L bis 20 L dieses Gemisches. Bevorzugt werden 8- 12 fach dieses Gemisches verwendet, besonders bevorzugt werden 9-11 fach dieses Gemisches verwendet, ganz besonders bevorzugt ist 10 fach.

Die Freisetzung der Zielverbindung (I) oder (Ia) erfolgt indem man das Diastereomerensalz (IVa oder IVb oder IV c oder IVd) in einem Lösungsmittelgemisch bei 0 bis 80°C, bevorzugt 20 bis 50°C vorlegt, anschließend durch Zugabe der organischen oder anorganischen Base (entweder in fester Form, oder als Lösung, bevorzugt in Wasser) einen pH-Wert von 6,9 bis 9,5 ,bevorzugt einen pH-Wert von 7,0 bis 8,0, besonders bevorzugt pH 7,5 einstellt. Die Base kann wahlweise sehr schnell (innerhalb weniger Minuten) oder auch sehr langsam (innerhalb mehrerer Stunden) zugegeben werden, wie beispielsweise in 5 Minuten bis zu 3 Stunden. Eine schnellere Zugabe wird auf jeden Fall bevorzugt, bevorzugt wird innerhalb von 5 min. bis 1 Stunde zu dosiert. Hierzu kann ein im Reaktor eingebautes pH-Meter dienen, mit dem man die Einstellung kontrolliert und die Base langsam zu dosiert. Man kann aber auch von Anfang an eine Festmenge an Base (fest oder in einem Lösungsmittel gelöst) zusetzen, die aufgrund von Erfahrungswerten sicherstellt, dass bevorzugt der gewünschte pH-Wert-Bereich erreicht wird. Ein solches Vorgehen wird in der Produktion besonders bevorzugt. Es hat sich als vorteilhaft erwiesen, wenn man nach erfolgter pH-Wert-Einstellung nochmals bei 10 bis 80°C, bevorzugt 20 bis 60°C, bevorzugt 40-60°C nachrührt. Die Nachrührzeit kann dabei 1 bis 10 Stunden, bevorzugt 2-5 Stunden, besonders bevorzugt 3-4 Stunden betragen. Anschließend lässt man auf 15 bis 25°C abkühlen, z.B. über eine Rampe und kann dann erneut 1 bis 64 Stunden nachrühren (die 64 Stunden dienen zur Demonstration der Robustheit des Verfahrens, siehe Beispiel 3b), bevorzugt wird aber zwischen 3 und 24 Stunden nachgerührt, in der Regel reichen aber 10 bis 20 Stunden.

Die Isolierung erfolgt nach dem Fachmann bekannten Methoden, wie zum Beispiel durch Filtration oder über eine Zentrifuge. Der so erhaltene Filterkuchen kann einmal, oder mehrmals mit einem Lösungsmittel, oder Lösungsmittelgemisch nachgewaschen werden. Anschließend erfolgt eine Trocknung unter Vakuum, bevorzugt < 100 mbar bei erhöhter Temperatur (50 bis 80°C, bevorzugt 50°C). Die Verwendung von Schleppgas hat sich in manchen Fällen als vorteilhaft erwiesen.

Es ist aber auch möglich das erhaltene Material in Ethanol oder Mischungen aus Wasser mit Ethanol vom Filter zu lösen und dann nach Justierung der Wasser/Ethanol-Menge im Eluat gleich in den nächsten Verfahrensschritt zu gehen (siehe unten). Dieses Vorgehen wird besonders großtechnisch bevorzugt, da man sich dadurch einen Trockenschritt sparen kann.

Mit der oben geschilderten Verfahrensweise gelingt es chemisch sehr reine Rohprodukte herzustellen. Der Enantiomeren-Überschuss der Rohprodukte (I) und (Ia) liegt in der Regel > 96,5 % e.e.

Im Rahmen des Up-Scalings hat es sich gezeigt, dass die vollständigere Abtrennung der Weinsäureester (IIIa) und (IIIb) aus den Rohprodukten (I) und (Ia) technisch nicht immer sehr einfach durchzuführen ist und man sich beim Gehalt dieser Komponenten eng am Limit der Spezifikation bewegt. Da die Anforderungen an die Spezifikation im finalen Wirkstoff sehr hoch sind (< 0,1 % (III a) im finalen Wirkstoff), hat es sich als vorteilhaft erwiesen in manchen Fällen einen weiteren Verfahrensschritt anzuschließend, der sicherstellt, dass der Gehalt an Weinsäureester (IIIa) auf unter 0,15%, vorzugsweise unter 0,1% und insbesondere unter 0,05 % reduziert wird und man reproduzierbar den finalen Wirkstoff spezifikationsgerecht erhält. Der Weinsäureester (IIIa), wird kaum, oder fast gar nicht in der finalen Kristallisation zum Reinprodukt abgereichert, sodass ein solcher nachgeschalteter Verfahrensschritt garantiert, dass eine robuste Fahrweise des Gesamt-Verfahrens gewährleistet wird, außerdem arbeitet man nahezu verlustarm. Dieser Verfahrensschritt, der sicherstellt, dass man Verbindung (IIIa) auf unterhalb von 0,15%, vorzugsweise unterhalb 0,1% und insbesondere unterhalb 0,05 % im Wirkstoff abreichert, ist ebenfalls Gegenstand der vorliegenden Erfindung. Das Verfahren zur Reduzierung von (IIIa) wird wie folgt durchgeführt. Man setzt hierzu das getrocknete oder vorteilhafterweise das noch feuchte Rohprodukt von (I) oder (Ia) ein. Hierzu wird erneut eine Base verwendet: Zur Entfernung von Weinsäureestern der Formel (III)a oder (IIIb) können anorganische und organische Basen verwendet werden. Im Falle von anorganischen Basen können Ammoniak, Natronlauge, Lithiumhydroxid, Kaliumhydroxid, Ammoniumcarbonat, Natriumcarbonat, Kaliumcarbonat, Lithiumcarbonat, Ammoniumhydrogencarbonat, Natriumhydrogencarbonat, Kaliumhydrogencarbonat, Natriumphosphat, Kaliumphosphat, Ammoniumphosphat eingesetzt. Bevorzugt wird jedoch Natriumhydroxid, Natriumphosphat oder Kaliumphosphat verwendet. Besonders bevorzugt wird Natriumphosphat oder Kaliumphosphat verwendet. Es ist wichtig zu betonen, dass die anorganischen Basen sowohl in wasserfreier Form, als auch in Form ihrer Hydrate eingesetzt werden können, so kann z.b. Natriumphosphat (wasserfrei) und Natriumphosphat-Hydrat erfolgreich verwendet werden. Als organische Base können aliphatische oder aromatische Basen, wie z.B. Triethylamin, Imidazol, N-Methylimidazol, Hünigbase, Pyridin, DBU verwendet werden.

Die Durchführung der Abreicherung von (IIIa) oder (IIIb) erfolgt in Gemischen von Wasser, mit mit Wasser mischbaren organischen Lösungsmitteln, wie Ethanol oder Isopropanol, 1,2-Ethandiol, Methoxyethanol oder Methanol, oder Isopropanol oder Aceton, bevorzugt wird aber Ethanol eingesetzt. Die Lösungsmittel können auch in der handelsüblichen vergällten Form, wie die beim Ethanol verwendeten Vergällungsmittel z.B. Toluol, Methylethylketon, Thiophen, Hexan eingesetzt werden, was aus kostentechnischen Gründen große Vorteile mit sich bringt. Es hat sich als vorteilhaft erwiesen Mischungen von Wasser und Ethanol zu verwenden, wobei Mischungen (Vol/Vol) im Bereich von Ethanol: Wasser = 30: 10 bis 10: 10 liegt. Bevorzugt wird aber eine Mischung von Ethanol: Wasser = 20: 10 verwendet. Die Mischung kann zuvor hergestellt werden, oder aber in situ, nach Vorlage aller Komponenten in einem Topf erzeugt werden. Das Lösungsmittelgemisch kann in 10 bis 20 fachem Überschuss bezogen auf das eingesetztes Diastereomerensalz (Iva), IVb, IVc oder IVd eingesetzt werden, also z.B. 1 kg in 10 L bis 20 L dieses Gemisches. Bevorzugt werden 10 - 14 fach dieses Gemisches verwendet, besonders bevorzugt werden 11-12 fach dieses Gemisches verwendet.

Die Durchführung der Abreicherung von (IIIa) oder (IIIb) erfolgt derart, daß man in einem Lösungsmittelgemisch, wie zuvor beschrieben bei 40 bis 80°C, bevorzugt 50 bis 70°C vorlegt, anschließend durch Zugabe der organischen oder anorganischen Base (entweder in fester Form, oder als Lösung, bevorzugt, Wasser) einen pH-Wert von 6,9 bis 9,5 ,bevorzugt einen pH-Wert von 7,5 bis 9,0 , besonders bevorzugt pH 8,5 einstellt. Die Base kann wahlweise sehr schnell (innerhalb weniger Minuten) oder auch sehr langsam (innerhalb mehrerer Stunden) zugegeben werden, wie zum Beispiel in 1 Minute bis zu 3 Stunden. Eine schnellere Zugabe wird auf jeden Fall bevorzugt, bevorzugt wird innerhalb von 1 min. bis 1 Stunde zu dosiert. Hierzu kann ein im Reaktor eingebautes pH-Meter dienen, mit dem man die Einstellung kontrolliert und die Base langsam zu dosiert. Man kann aber auch von Anfang an eine Festmenge an Base (fest oder in einem Lösungsmittel gelöst) zusetzen, die aufgrund von Erfahrungswerten sicherstellt, dass bevorzugt der gewünschte pH-Wert-Bereich erreicht wird. Ein solches Vorgehen wird in der Produktion am meisten bevorzugt. Es hat sich als vorteilhaft erwiesen, wenn man nach erfolgter pH-Wert-Einstellung nochmals bei 40-80°C, bevorzugt 50 - 75 °C, bevorzugt 60-70°C nachrührt. Die Nachrührzeit kann dabei 1 bis 24 Stunden, bevorzugt 2-10 Stunden, besonders bevorzugt 2-4 Stunden betragen. Anschließend wird das organische Lösungsmittel weitgehend abdestilliert, dies kann bei Normaldruck erfolgen oder schonender unter reduziertem Druck. Sobald das organische Lösungsmittel weitgehend ab destilliert ist lässt man auf 15-25°C abkühlen, z.B. über eine Rampe und kann dann erneut 1 bis 64 Stunden nachrühren (die 64 Stunden dienen zur Demonstration der Robustheit des Verfahrens, siehe Beispiel 3b), bevorzugt wird aber zwischen 1 und 24 Stunden nachgerührt, in der Regel reichen aber 1-5 Stunden.

Die Isolierung erfolgt nach dem Fachmann bekannten Methoden, wie zum Beispiel durch Filtration oder über eine Zentrifuge. Der so erhaltene Filterkuchen kann einmal, oder mehrmals mit einem Lösungsmittel, oder Lösungsmittel Gemisch nachgewaschen werden. Anschließend erfolgt eine Trocknung unter Vakuum, bevorzugt < 100 mbar bei erhöhter Temperatur (50 bis 80°C, bevorzugt 50°C). Die Verwendung von Schleppgas hat sich in manchen Fällen als vorteilhaft erwiesen.

Es ist auch möglich vor dem Abdestillieren des Lösungsmittel den pH-Wert mit eine organischen Säure, wie zum Beispiel Ameisensäure, Zitronensäure, Essigsäure oder anorganische Säure, wie zum Beispiel Salzsäure, Schwefelsäure, Phosphorsäure oder einem sauren Salz, wie z.B Natriumdihydrogenphosphat, Kaliumhydrogensulfat oder Natriumhydrogensulfat auf pH 5 - 7 zurück zu stellen und dann wie oben beschrieben fortzufahren.

Mit der oben geschilderten Verfahrensweise gelingt es chemisch sehr reine Rohprodukte herzustellen. Der Enantiomeren-Überschuss der Rohprodukte (I) und (Ia) liegt in der Regel > 97 % e.e., Der Gehalt an (IIIa) oder (IIIb) ist unter 0,15%, vorzugsweise unter 0,1% und insbesondere unter 0,05 % reduziert.

Es hat sich als vorteilhaft erwiesen ein so erhaltenes Rohprodukt der Formel (I) oder (Ia) noch einer nachträglichen Kristallisation zu unterziehen um sowohl die chemische als auch vor allem die optische Reinheit zu verbessern, da die Enantiomeren-Überschüsse der Rohprodukte in der Regel > 97 % e.e. liegen. Dazu wurde ein finales Kristallisations-Verfahren entwickelt: Hierzu wird aus GMP-technischen Gründen das Rohprodukt (I) oder (Ia) in Ethanol gelöst, (gegebenenfalls durch Erwärmung) und anschließend einer Partikelfiltration unterzogen, bevorzugt wird Branntwein oder mit Toluol vergälltes Ethanol eingesetzt. In manchen Fällen, abhängig vom technischen Equipment, kann man die Kristallisation auch unter Druck, bei erhöhter Temperatur fahren (Vorteil: weniger Lösungsmittel), es kann aber auch aus wässrigen Ethanol-Lösungen umkristallisiert werden (unter Druck oder bei Normaldruck). Dann wird unter Normaldruck, bzw. unter reduziertem Druck auf ca. 3 bis 6 fach Volumen eingeengt, bevorzugt 4 bis 5 fach, dabei kristallisiert das Produkt aus. In manchen Fällen, wenn z.B. große Volumina über lange Zeiten abdestilliert werden müssen hat es sich als vorteilhaft erwiesen unter reduziertem Druck abzudestillieren, wobei man die Innentemperatur niedrig hält um Zersetzungen und Nebenprodukt-Bildung zu vermeiden. Nach Beendigung der Destillation wird auf 0°C abgekühlt und anschließend die Kristalle isoliert und im Vakuum bei 40 bis 50 °C getrocknet. Die Ausbeuten sind in der Regel > 88% d. Theorie. Die erzielte chemische, Reinheit und der Gehalt entsprechen den Kriterien für Handelsprodukte nach ICH-Guideline. Restlösungsmittel, in dem Fall Ethanol, ist in der Regel bei den Technikumsansätzen < 0,05 %. Die optische Reinheit beträgt » 99 % e.e.

Als besonders bevorzugtes Verfahren, insbesondere für die großtechnische Durchführung setzt man (+)-Dibenzoyl-Weinsäure (V) ein, es kann sowohl die wasserfreie Form, als auch das Hydrat eingesetzt werden:

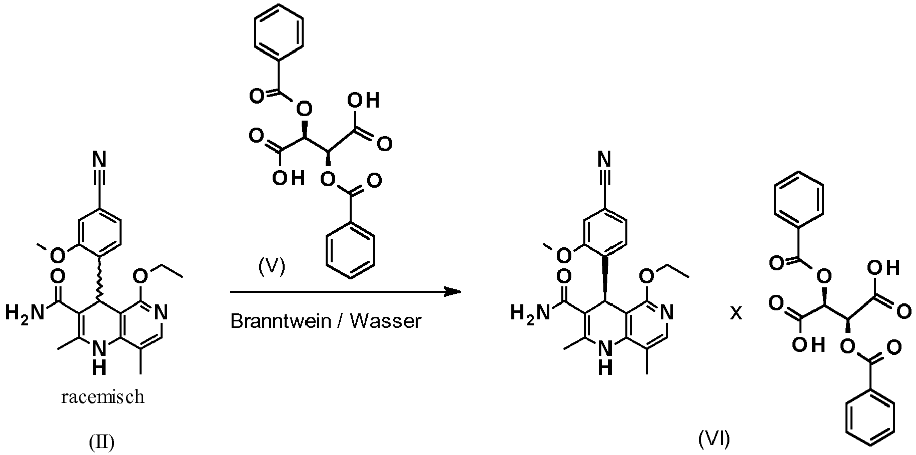

Die Racematspaltung wird bevorzugt in einem Branntwein-Wasser-Gemisch durchgeführt. Die anschließende Freisetzung von Finerenone, roh 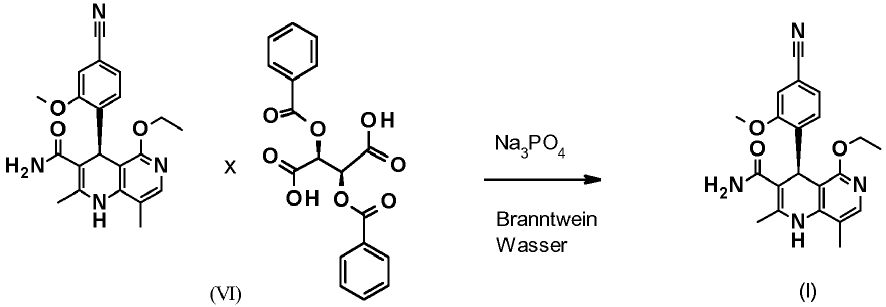 erfolgt bevorzugt in einem Branntwein-Wasser-Gemisch unter Verwendung von Natriumphosphat als Base. In Fällen, in denen eine Umarbeitung nötig ist, wenn der Anteil an (+)-Dibenzoyl-Weinsäure (V) > 0,15 % beträgt 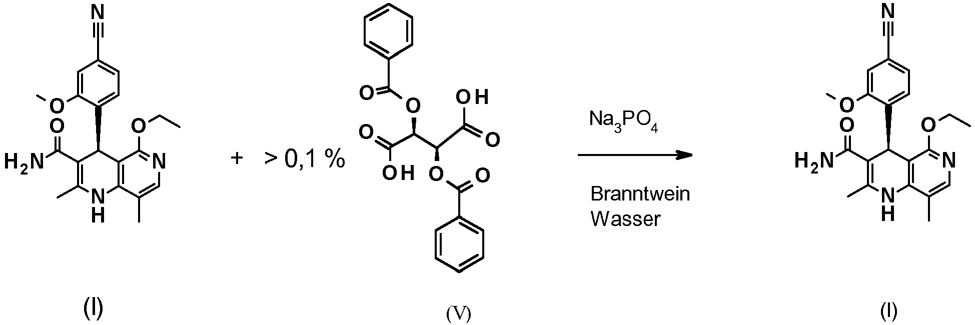 erfolgt die Umarbeitung bevorzugt in einem Branntwein-Wasser-Gemisch unter Verwendung von Natriumphosphat als Base. Die finale Kristallisation zu Finerenone, rein erfolgt bevorzugt in Branntwein als Lösungsmittel. Dabei wird eine Reinheit von 0,15% bevorzugt 0,1% besonders bevorzugt unter 0,05% erreicht.

Es ist auch möglich das Ziel-Enantiomer aus der Mutterlauge zu isolieren. Hier wird zuerst das entsprechende Diastereomeren-Salz (IVa), (IVb), (IVc) oder (IVd) entweder von (I) oder (Ia) hergestellt, dann durch Abfiltrieren isoliert und anschließend die Mutterlauge, die dann den jeweiligen Antipoden enthält durch Zugabe einer Base, wie z.B. Ammoniak, Natronlauge, Lithiumhydroxid, Kaliumhydroxid, Ammoniumcarbonat, Natriumcarbonat, Kaliumcarbonat, Lithiumcarbonat, Ammoniumhydrogencarbonat, Natriumhydrogencarbonat, Kaliumhydrogencarbonat, Natriumphosphat, Kaliumphosphat, Ammoniumphosphat , bevorzugt Natriumhydroxid, Natriumphosphat und Kaliumphosphat, besonders bevorzugt Natriumphosphat und Kaliumphosphat der pH-Wert auf pH > 7 eingestellt, bevorzugt ist pH 7,5. Anschließend wird das organische Solvens, bevorzugt Ethanol abdestilliert, entweder bei Normaldruck oder schonender unter reduziertem Druck, dabei fällt der entsprechende Antipode aus. Das Produkt wird abfiltriert, mit Wasser oder Wasser/LösungsmittelGemischen gewaschen und getrocknet. Eine entsprechende finale Kristallisation aus Branntwein, wie z.B. in Beispiel 1c beschrieben liefert die Verbindungen (I) und (Ia) in entsprechend reiner Form.

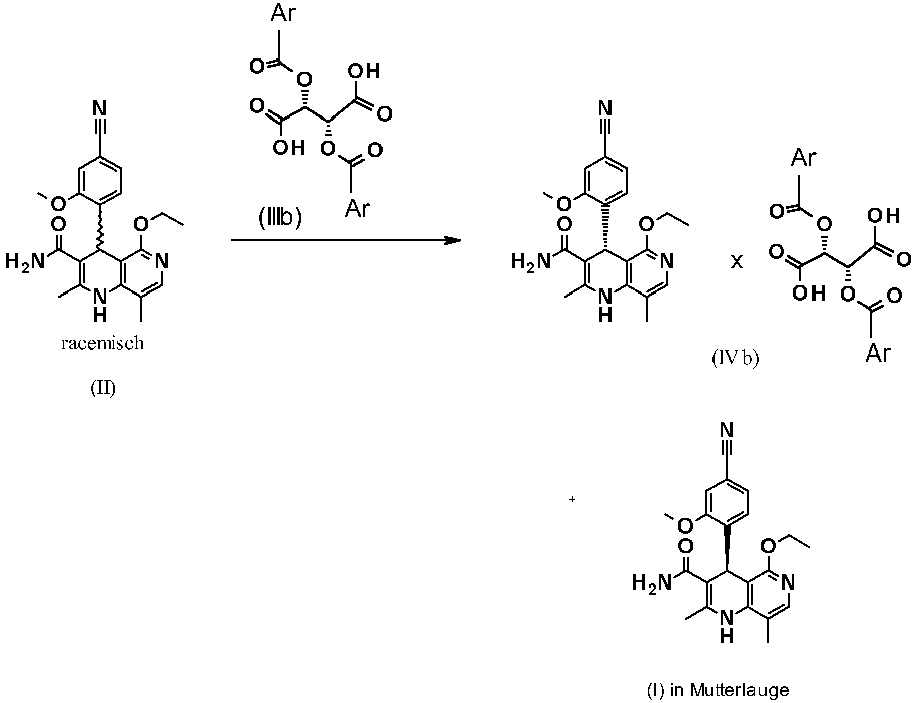

Ein weiterer Gegenstand der vorliegenden Anmeldung ist daher Finerenone (I) mit einem Dibenzoylweinsäuregehalt ≤ 0,15% dadurch erhalten, dass man das Racemat (II) 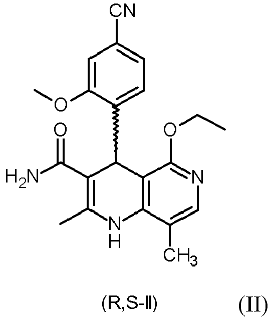 mit Dibenzoylweinsäure der Formel (V) 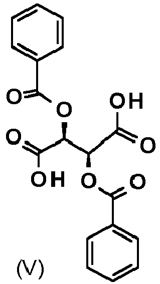 in einem Branntwein/ Wasser Gemisch zu dem Diastereomerensalz (VI) 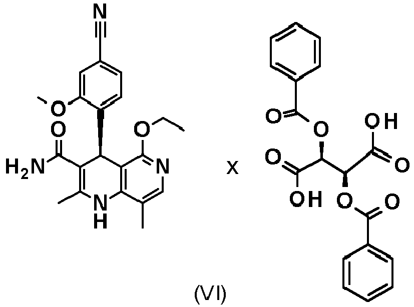 umsetzt und anschließend Finerenone (I) 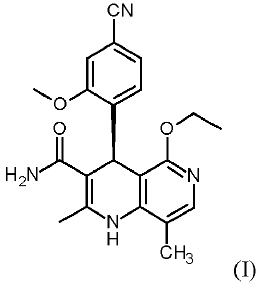 unter Einsatz von Natriumphosphat ebenfalls in einem Branntwein/ Wasser Gemisch freisetzt und anschliessend erneut mit Natriumphosphat in Branntwein/ Wassergemisch umsetzt und dann reines Finerenone mit einem Dibenzoylweinsäuregehalt ≤ 0,15% auskristallisiert; in einer besonders bevorzugten Ausführungsform erhält man Finerenone mit einem Dibenzoylweinsäuregehalt ≤ 0,1%, insbesondere bevorzugt unter 0,05% Dibenzoylweinsäuregehalt.

### Experimenteller Teil

### Abkürzungen und Akronyme:

- **EtOH**: **Ethanol**
- **DB Weinsäure**: **Dibenzoylweinsäure**
- **DMSO**: **Dimethylsulfoxid**
- **d.Th**: **Der Theorie (bei Ausbeute)**
- HPLC: Hochdruck-, Hochleistungsflüssigchromatographie
- **1H-NMR**: **1H Kernresonanzspektrometrie**
- **IT**: **Innentemperatur**
- **MS**: **Massenspektrometrie**
- **RT**: **Retention Time**
- **RRT**: **Relative Retention Time**
- **TFA**: **Trifluoressigsäure**

- **TM**: **Manteltemperatur**
- **XRPD**: **X-ray Powderdiffraction (Pulverdiffraktometer)**
- **Branntwein**: **Ethanol mit 2% Toluol vergällt**

### Beispiele

Die nachfolgende Tabelle 3 gibt die Strukturen der in der HPLC wiedergefundenen Verbindungen wieder. Die Zuordnung der Retentionszeiten im HPLC ist unten angegeben.

**Tabelle 3**

| **Finerenone (I)** |
|---|
| 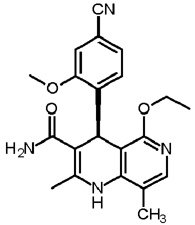 |
| Verunreinigung A |
| 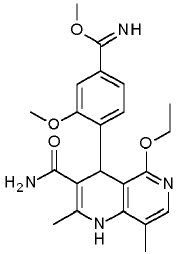 |
| Verunreinigung B |
| 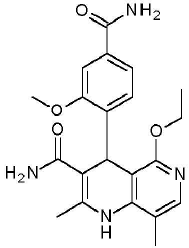 |
| Verunreinigung C (unbekannte Struktur, immer signifikant unter 0,1 %) |
| Verunreinigung D |
| 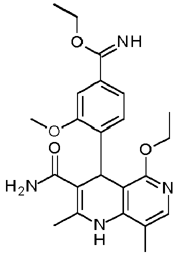 |
| Verunreinigung E |
| 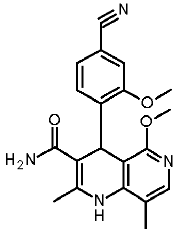 |
| Verunreinigung F |
| 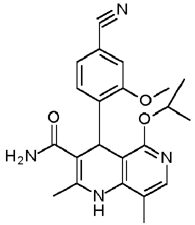 |
| Verunreinigung G |
| 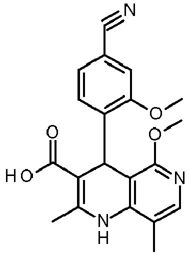 |
| Verunreinigung H |
| 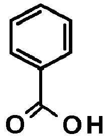 |
| Verunreinigung I |
| 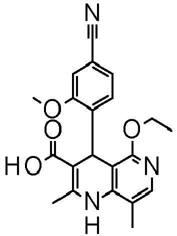 |
| Verunreinigung J |
| 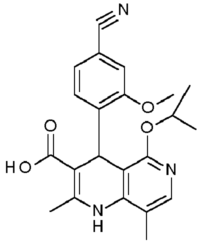 |
| Verunreinigung K |
| 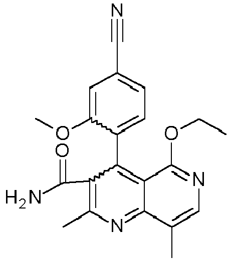 |
| Verunreinigung N (III) |
| 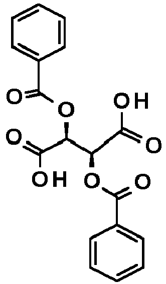 |

### 1) Analytische Methode zur Prüfung des Gehalts an Verunreinigungen und Enantiomerenreinheit auf der Stufe Di-Benzoyl-Weinsäure

| **Gehalt und organische Verunreinigungen** | | | | RT(min) | RRT |
|---|---|---|---|---|---|
| | Di-Benzoyl-Weinsäure | | | Ca. 11,1 | 1,00 |
| | 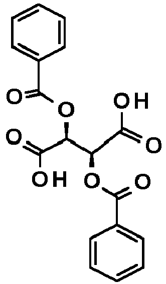 | | | | |
| | | | | | |
| | Mono- Benzoyl- Weinsäure | | | Ca. 5,1 | 0,46 |
| | 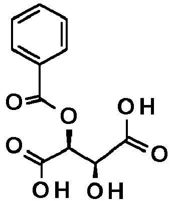 | | | | |
| | | | | | |
| | Benzoesäure | | | Ca. 7,6 | 0,69 |
| | 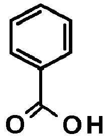 | | | | |
| | | | | | |
| **Gerät:** | Ultra-Hochleistungsflüssigkeitschromatograph (mit einem Druckbereich bis 1200 bar mit thermostatisiertem Säulenofen, und UV-Detektor | | | | |
| | | | | | |
| **Säule :** | YMC Triart C8 | | | | |
| | Länge: 100 mm, Innendurchmesser: 3,0 mm, Korngröße: 1,9 µm | | | | |
| | Maxdruck: 1000 bar | | | | |
| | | | | | |
| **Bedingungen:** | 20°C; 0,50 mL/min; 1,7µL(10°C); 240nm/6nm | | | | |
| | | | | | |
| **Elutionsmittel:** | A: 0,1% TFA in Wasser; B: Acetonitril | | | | |
| | | | | | |
| **Gradient:** | Zeitpunkt (min) | A (%) | B (%) | | |
| | 0,0 | 90,0 | 10,0 | | |
| | 15,0 | 35,0 | 65,0 | | |
| | 16,0 | 20,0 | 80,0 | | |
| | 20,0 | 20,0 | 80,0 | | |
| | | | | | |
| **Enantiomerenreinheit:** | | | | RT(min) | RRT |
| | (+)-Di-Benzoyl-Weinsäure | | | 2,1 | 1,00 |
| | (-)-Di-Benzoyl-Weinsäure | | | 3,9 | 1,86 |
| | | | | | |
| **Gerät:** | Hochleistungsflüssigkeitschromatograph mit thermostatisiertem Säulenofen und UV-Detektor | | | | |
| | | | | | |
| **Säule :** | **Chiralpak IC** | | | | |
| | Länge: 250 mm, Innendurchmesser: 4,6 mm, Korngröße: 5,0 µm | | | | |
| | Maxdruck: 300 bar | | | | |
| | | | | | |
| **Bedingungen:** | 40 °C; 2,0 mL/min ; 5 µL; 234nm/6nm | | | | |
| **Elutionsmittel:** | A: Heptan; B: 0,1% TFA in Ethanol | | | | |
| | | | | | |
| **Isokratisch:** | A(%) 80: B (%) 20 | | | | |

### 2) Analytische Methode zur Prüfung des Gehalts an Verunreinigungen und Enantiomerenreinheit auf der Stufe des Diastereomerensalzes

| **Gehalt und organische Verunreinigungen** | | RT(min) | RRT |
|---|---|---|---|
| | **Finerenone (I)** | **6,2** | **1,00** |
| | Verunreinigung A | 3,3 | 0,53 |
| | Verunreinigung B | 3,7 | 0,60 |
| | VerunreinigungC | 3,9 | 0,62 |
| | Verunreinigung D | 4,4 | 0,70 |
| | Verunreinigung E | 5,5 | 0,89 |
| | Verunreinigung F | 6,8 | 1,10 |
| | Verunreinigung G | 7,2 | 1,17 |
| | Verunreinigung H | 7,7 | 1,25 |
| | Verunreinigung I | 7,8 | 1,27 |
| | Verunreinigung J | 8,4 | 1,36 |
| | Verunreinigung K | 10,4 | 1,69 |
| | Verunreinigung N | 11,1 | 1,80 |
| | | | |
| **Gerät:** | Ultra-Hochleistungsflüssigkeitschromatograph (mit einem Druckbereich bis 1200 bar mit thermostatisiertem Säulenofen, und UV-Detektor | | |
| | | | |
| **Säule :** | YMC Triart C8 | | |
| | Länge: 100 mm, Innendurchmesser:3,0 mm, Korngrösse: 1,9 µm | | |
| | Maxdruck: 1000 bar | | |
| | | | |
| | | | |
| **Bedingungen:** | 20°C; 0,50 mL/min; 3,5µL(10°C); 242 nm/6nm | | |
| | | | |
| **Elutionsmittel:** | A: 0,1% TFA in Wasser; B: Acetonitril | | |
| | | | |
| **Gradient:** | Zeitpunkt (min) | A (%) | B (%) |
| | | | |
| | 0,0 | 90,0 | 10,0 |
| | 15,0 | 35,0 | 65,0 |
| | 16,0 | 20,0 | 80,0 |
| | 20,0 | 20,0 | 80,0 |

| **Enantiomerenreinheit:** | | RT(min) | RRT |
|---|---|---|---|
| | **Finerenone (I)** | **5,34** | **1,00** |
| | (Ia) | 6,14 | **1,15** |
| | | | |
| **Gerät:** | Hochleistungsflüssigkeitschromatograph mit thermostatisiertem Säulenofen und UV-Detektor | | |
| | | | |
| **Säule :** | **Lux 3µm i-Cellulose-5** | | |
| | Länge: 150 mm, Innendurchmesser: 4,6 mm, Korngrösse: 3,0 µm | | |
| | Maxdruck: 300 bar | | |
| | | | |
| **Bedingungen:** | 40 °C; 1,0 mL/min ; 10 µL(20°C); 252nm/6nm | | |
| | | | |
| **Elutionsmittel:** | A: 20mmol Ammoniumacetatpuffer pH 9,0 (1,54g Ammoniumacetat in 1L Milli-Q-Wasser und mit Ammoniak auf pH 9,0 eingestellt) ; B: Acetonitril | | |
| | | | |
| **Isokratisch:** | A(%) 50: B (%) 50 | | |

### 3) Analytische Methode zur Prüfung des Gehalts an Verunreinigungen und Enantiomerenreinheit auf der Stufe Finerenone, roh (I).

| **Gehalt und organische Verunreinigungen** | | RT(min) | RRT | |
|---|---|---|---|---|
| | **Finerenone (I)** | **6,2** | **1,00** | |
| | Verunreinigung A | 3,3 | 0,53 | |
| | Verunreinigung B | 3,7 | 0,60 | |
| | Verunreinigung C | 3,9 | 0,62 | |
| | Verunreinigung D | 4,4 | 0,70 | |
| | Verunreinigung E | 5,5 | 0,89 | |
| | Verunreinigung F | 5,6 | 0,91 | |
| | Verunreinigung G | 6,8 | 1,10 | |
| | Verunreinigung H | 7,6 | 1,23 | |
| | Verunreinigung K | 10,4 | 1,68 | |
| | Verunreinigung N | 11,1 | 1,79 | |
| | | | | |
| **Gerät:** | Ultra-Hochleistungsflüssigkeitschromatograph (mit einem Druckbereich bis 1200 bar mit thermostatisiertem Säulenofen und UV-Detektor | | | |
| | | | | |
| **Säule :** | YMC Triart C8 | | | |
| | Länge: 100 mm, Innendurchmesser:3,0 mm, Korngrösse: 1,9 µm Maxdruck: 1000 bar | | | |
| | | | | |
| **Bedingungen:** | 20°C; 0,50 mL/min; 1,7µL(10°C); 252 nm/6nm und 230nm/6nm für die Auswertung von DB-Weinsäure | | | |
| **Elutionsmittel:** | A: 0,1% TFA in Wasser; B: Acetonitril | | | |
| **Gradient:** | Zeitpunkt (min) | A (%) | B (%) | |
| | 0,0 | 90,0 | 10,0 | |
| | 15,0 | 35,0 | 65,0 | |
| | 16,0 | 20,0 | 80,0 | |
| | 20,0 | 20,0 | 80,0 | |
| | | | | |
| **Enantiomerenreinheit:** | | | RT(min) | RRT |
| **Methode A** | Finerenone (I) | | Ca. 11 | 1,00 |
| | (Ia) | | Ca. 9 | 0,82 |
| Gerät: | Hochleistungsflüssigkeitschromatograph mit thermostatisiertem Säulenofen und UV-Detektor | | | |
| **Säule :** | **Chiralpak IA** Länge: 250 mm, Innendurchmesser: 4,6 mm, Korngrösse: 5,0 µm Maxdruck: 300 bar | | | |
| **Bedingungen:** | 40 °C; 0,8 mL/min ; 5 µL(20°C); 255nm/6nm | | | |
| **Elutionsmittel:** | A: Acetonitril; B: Methyl-tert-butyl-ether (MTBE) | | | |
| **Isokratisch:** | A(%) 90: B (%) 10 | | | |
| | | | | |
| **Enantiomerenreinheit:** | | | RT(min) | RRT |
| **Methode B** | Finerenone (I) | | 5.7 | 1,00 |
| | Enantiomer (Ia) | | 6.8 | 1.19 |
| | | | | |
| **Gerät/Detektor:** | Hochleistungsflüssigkeitschromatograph mit thermostatisiertem Säulenofen, UV Detektor und Datenauswertesystem | | | |
| **Messwellenlänge:** | 252 nm | | | |
| **Ofentemperatur:** | 40°C | | | |
| **Säule:** | **Chiralpak IC** | | | |
| **Länge:** | 150 mm, Innendurchmesser: 4.6 mm, Korngrösse: 3 µm | | | |
| **Mobile Phase:** | A: 50% Puffer 20mM Ammoniumacetat pH 9 | | | |
| | B: 50% Acetonitril | | | |
| **Fluss:** | 1 mL/min | | | |
| **Laufzeit:** | 8 min | | | |
| **Equilibrierung:** | nicht nötig, isokratisch | | | |
| **Probenlösungsmittel:** | Fließmittel | | | |
| **Prüflösung:** | ca. 0,5 mg/mL der Substanz Racemat, mit Probenlösungsmittel lösen | | | |
| **Vergleichslösung:** | Es wird eine Vergleichslösung analog zur Prüflösung hergestellt | | | |
| **Inj ektionsvolumen:** | 10 µL | | | |

Die in den nachfolgenden Beispielen angegebenen Messwerte zur Enantiomeren-Bestimmung wurden alle nach Methode B bestimmt. Einige Werte, vor allen die in der Technikums-Anlage hergestellten Batche wurden mit Methode A zum Vergleich nachgemessen und lieferten vergleichbare Ergebnisse.

Die in den nachfolgenden Beispielen angegebenen HPLC-Analysendaten bzgl. Reinheit und Gehalt für das Endprodukt Finerenone, rein (I) beziehen sich nur auf Verunreinigungen, die mit > 0,05 % im Produkt vorhanden sind. Das ist im Wesentlichen die Verunreinigung E. Alle anderen Verunreinigungen, die in der oben aufgeführten Tabelle gezeigt sind, sind in der Regel < 0,05 %. Die Struktur derartiger Verunreinigungen wurde durch Isolierung aus angereicherten Mutterlaugen bestimmt.

### Beispiel 1

Laboransatz unter Verwendung von wasserfreier (+)-O,O-Dibenzoyl-D-Weinsäure (III)

### Beispiel 1a

### Tartrat-Salz (IV) Herstellung

250 g (660,616 mmol) Racemat von Finerenone (II) wurden in 3500 ml eines Gemisches bestehend aus Ethanol, Toluol vergällt/Wasser = 75:25 (v/v) bei Raumtemperatur (ca. 23°C) vorgelegt. Es wurden 130,2 g (363,339 mmol) (+)-O,O-Dibenzoyl-D-Weinsäure (III) über einen Feststofftrichter zugegeben und dann mit 250 ml eines Gemisches bestehend aus Ethanol, Toluol vergällt/Wasser = 75:25 (v/v) nachgespült. Die so erhaltene Suspension wurde innerhalb 0,75 Stunden auf 75°C Innentemperatur erwärmt und anschließend 3,0 Stunden bei dieser Temperatur nachgerührt. Anschließend wurde innerhalb von 5,0 Stunden über eine Abkühlrampe ab 23°C abgekühlt und über Nacht (ca. 16 Stunden) bei dieser Temperatur nachgerührt. Die Suspension wurde über eine Fritte abfiltriert und einmal mit 250 ml eines Gemisches bestehend aus Ethanol, Toluol vergällt/Wasser = 75:25 (v/v) nachgewaschen. Feuchtausbeute: 334,7 g. Das Feuchtprodukt wurde anschließend über Nacht (ca. 16 Stunden) bei 50°C unter Vakuum (< 100 mbar) getrocknet. Ausbeute: 250,2 g (100,08 % d.Th.) eines farblosen kristallinen Pulvers

### Analytische Ergebnisse:

| Finerenone (I) | 47,2 % Gewichtsprozent (HPLC) |
|---|---|
| Enantiomerenüberschuss | 97,68 % e.e. |
| Größte unbekannte Nebenkomponente bei RT 5,606 Min. | 0,47 % |
| Restlösungsmittel: | |
| EtOH | 2,24 % |
| Toluol | 0,0 % |

MS (EIpos): m/z = 379 [M+H]⁺
¹H-NMR (400 MHz, DMSO-d₆): δ = 1.05 (t, 3H), 2.12 (s, 3H), 2.18 (s, 3H), 3.82 (s, 3H), 3.99-4.07 (m, 2H), 5.39 (s, 1H), 5,89 (s, 2H), 6.60-6.84 (m (breites Signal), 2H), 7.14 (d, 1H), 7.28 (dd, 1H), 7.37 (d, 1H), 7.55 (s, 1H), 7,61 (t, 4H), 7.69 (s, 1H), 7,75 (t, 2H), 8,04 (d, 4H), 12,50-15,40 (sehr breites Signal, 2H) und Signal vom Lösungsmittel DMSO und erhöhtes Wasser-Signal: δ = 2,5-2,6, sowie kleine Peaks bei δ = 3,40 - 3,50 (q) und δ = 1,05- 1,10 (t), überlagertes Signale aus Restlösungsmittel Ethanol.

### Beispiel 1b

### Herstellung Rohprodukt (I)

248 g der in Beispiel 1a hergestellten Verbindung (IV) wurde bei Raumtemperatur in 2480 ml eines Gemisches bestehend aus Ethanol, Toluol vergällt/Wasser = 20:80 (v/v) suspendiert (es wurde ein pH-Wert von pH=4 gemessen). Anschließend wurden 819,6 g einer wässrigen Natriumphosphat-Lösung (100g Natriumphosphat gelöst in 1000 ml Wasser) innerhalb von 60 Minuten zu getropft und der pH-Wert auf pH=7,2 eingestellt. Es wurde 50 Minuten bei 23°C nachgerührt (pH=7,1). Anschließend wurden 98,3 g einer wässrigen Natriumphosphat-Lösung (100g Natriumphosphat gelöst in 1000 ml Wasser) innerhalb von 10 Minuten zugetropft und der pH-Wert auf pH=7,5 eingestellt. Es wurde innerhalb einer Stunde auf 50°C Inntemperatur erwärmt und 3,0 Stunden bei dieser Temperatur nach gerührt. Es wurde innerhalb einer Stunde auf 22°C abgekühlt und eine Stunde bei dieser Temperatur nachgerührt. Das Kristallisat wird über eine Fritte abfiltriert und einmal mit 200 ml und einmal mit 100 ml eines Gemisches bestehend aus Ethanol, Toluol vergällt/Wasser = 20:80 (v/v) und zweimal mit 200 g Wasser nachgewaschen. Feuchtausbeute: 263,4 g. Das Feuchtprodukt wurde anschließend übers Wochenende (> 48 Stunden) bei 50°C unter Vakuum (< 100 mbar) getrocknet. Ausbeute: 116,9 g (93,52 % d.Th.) eines farblosen kristallinen Pulvers.

### Analytische Ergebnisse:

| Finerenone (I) | Reinheit: 99,86% Fläche (HPLC); Gehalt: 100,0 Gew. % |
|---|---|
| Enantiomerenüberschuss | 97,02 % e.e. |
| Größte Nebenkomponente Verunreinigung E | 0,07 % |
| Restlösungsmittel: | |
| EtOH | 0,19% |
| Toluol | 0,13 % |
| Wasser (Karl-Fischer) | 0,042 % |

MS (EIpos): m/z = 379 [M+H]⁺
¹H-NMR (400 MHz, DMSO-d₆): δ = 1.05 (t, 3H), 2.12 (s, 3H), 2.18 (s, 3H), 3.82 (s, 3H), 3.99-4.07 (m, 2H), 5.37 (s, 1H), 6.60-6.84 (m (breites Signal), 2H), 7.14 (d, 1H), 7.28 (dd, 1H), 7.37 (d, 1H), 7.55 (s, 1H), 7.69 (s, 1H) und Signal vom Lösungsmittel DMSO und signifikant erhöhtes Wasser-Signal: δ = 2,5-2,6, sowie ein sehr kleiner Peak bei δ = 3,38 (nicht zuzuordnen)

### Beispiel 1c

### Herstellung Reinprodukt (I)

116,0 g des in Beispiel 1b hergestellten Rohproduktes (I) wurden in 2330 ml Ethanol, Toluol vergällt suspendiert und anschließend wurde zum Rückfluss erhitzt. Das Produkt ging dabei in Lösung. Es wurde eine Stunde bei dieser Temperatur nachgerührt. Die Lösung wurde über einen beheizten Druckfilter (T=75°C) abfiltriert, der Druckfilter anschließend mit 30 ml Ethanol, Toluol vergällt nachgespült. Es wurde anschließend das Lösungsmittel soweit ab destilliert (ca. 1920 ml wurden ab destilliert) bis ein Endvolumen von ca. 4 fach (bzg. auf eingesetzte Substanz: 116 g x 4 ∼ 484 ml) erreicht wurde. Anschließend wurde auf 23°C Innentemperatur abgekühlt (Dauer ca. 1,5 bis 2 Stunden). Dann wurde 2 Stunden bei 3°C Innentemperatur gerührt. Das Produkt wurde abfiltriert und einmal mit 100 ml Ethanol, Toluol vergällt nachgewaschen. Feuchtausbeute: 124 g. Das Feuchtprodukt wurde bei 50°C übers Wochenende (> 48 h) unter Vakuum (< 100 mbar) getrocknet. Ausbeute: 112,6 g (97,07 % d.Th.) eines farblosen kristallinen Pulvers, feine Nadelkristalle.

### Analytische Ergebnisse:

| Finerenone (I) | Reinheit: 99,86 Fläche (HPLC); Gehalt: 99,5 Gew. % |
|---|---|
| Enantiomerenüberschuss | 100 % e.e. |
| Größte Nebenkomponente Verunreinigung E | 0,07 % |
| Restlösungsmittel: | |
| EtOH | 0,05 % |
| Toluol | 0,00 % |
| Wasser (Karl-Fischer) | 0,00 % |

MS (EIpos): m/z = 379 [M+H]⁺
¹H-NMR (400 MHz, DMSO-d₆): δ = 1.05 (t, 3H), 2.12 (s, 3H), 2.18 (s, 3H), 3.82 (s, 3H), 3.99-4.07 (m, 2H), 5.37 (s, 1H), 6.60-6.84 (m (breites Signal), 2H), 7.14 (d, 1H), 7.28 (dd, 1H), 7.37 (d, 1H), 7.55 (s, 1H), 7.69 (s, 1H) und kleine Signale vom Lösungsmittel DMSO und Wasser bei δ = 2,5-2,6 sowie ein sehr kleiner Peak bei δ = 3,38 (nicht zuzuordnen)
Modifikation: Mod A (gemäß der Definition in WO2016/016287 A1)

### Beispiel 2

Laboransatz unter Verwendung von (+)-O,O-Dibenzoyl-D-Weinsäure-Hydrat (III)

### Beispiel 2a

### Tartrat Herstellung

350,0 g Racemat (II) wurden bei Raumtemperatur vorgelegt und anschließend 3112 g Branntwein zu gegeben. Dann wurde 1200 g Wasser zugesetzt. Es wurden 191,4 g % (+)-O,O-Dibenzoyl-D-Weinsäure-Monohydrat (III) über einen Feststofftrichter zugegeben und anschließend mit 113 g Wasser nachgespült. Die Suspension wurde innerhalb einer Stunde auf 75°C Innentemperatur erwärmt und anschließend 3 Stunden bei 75°C gerührt. Anschließend wurde innerhalb von 5,0 Stunden über eine Abkühlrampe ab 23°C abgekühlt und über Nacht (ca. 18 Stunden) bei dieser Temperatur nachgerührt. Die Suspension wurde über eine Fritte abfiltriert und zweimal mit einem Gemisch bestehend aus 332,3 g Branntwein und 140,2 g Wasser nachgewaschen nach gewaschen. Feuchtausbeute: 487,6 g. Das Feuchtprodukt wurde anschließend übers Wochenende(> 48 Stunden) bei 50°C unter Vakuum (< 100 mbar) getrocknet. Ausbeute: 351,0 g (100,29 % d.Th.) eines farblosen kristallinen Pulvers.

| Finerenone (I) | Gehalt: 47,92 Gew. % |
|---|---|
| Enantiomerenüberschuss | 97,84 % e.e. |
| Größte Nebenkomponente bei RT 5,86 Min. | 0,22 % |
| Restlösungsmittel: | |
| EtOH | 2,594 % |
| Toluol | 0,003 % |

In analoger Weise, wie in Beispiel 1b und 1c beschrieben kann aus diesem Tartrat-Salz das reine Finerenone (I) hergestellt werden.

### Beispiel 3

Herstellung von 3 Batchen reinen Finerenone (I) im technischen Labor, ausgehend von 2 kg Racemat (II).

### Beispiel 3a

### Tartrat Herstellung (IV)

2,00 kg (5,285 mol) racemisches Finerenone (II) wurden in 28,0 L eines Gemisches bestehend aus Ethanol, Toluol vergällt/Wasser = 75:25 (v/v) bei Raumtemperatur (ca. 23°C) vorgelegt. Es wurden 1,042 kg (2,907 mol) (+)-O,O-Dibenzoyl-D-Weinsäure (III) über einen Feststofftrichter zugegeben und dann mit 2 L eines Gemisches bestehend aus Ethanol, Toluol vergällt/Wasser = 75:25 (v/v) nachgespült. Die so erhaltene Suspension wurde innerhalb 45 Minuten auf 75°C Innentemperatur erwärmt und anschließend 3,0 Stunden bei dieser Temperatur nachgerührt. Anschließend wurde innerhalb von 5,0 Stunden über eine Abkühlrampe ab 23°C abgekühlt und über Nacht (ca. 16 Stunden) bei dieser Temperatur nachgerührt. Die Suspension wurde über eine Fritte abfiltriert und einmal mit 2 L eines Gemisches bestehend aus Ethanol, Toluol vergällt/Wasser = 75:25 (v/v) nachgewaschen und 10 Minuten trocken gesaugt. Feuchtausbeute: 2,69 kg. Das Feuchtprodukt wurde anschließend bei 50°C unter Vakuum (< 100 mbar) bis zur Massenkonstanz getrocknet (nach ca. 17 Stunden massenkonstant. Ausbeute: 2,009 kg (∼ 100 % d.Th.) eines farblosen kristallinen Pulvers

### Beispiel 3b

### Herstellung Rohprodukt (I)

2,006 kg der in Beispiel 3a hergestellten Verbindung (IV) wurde bei Raumtemperatur (ca. 23°C) in 20,0 L eines Gemisches bestehend aus Ethanol, Toluol vergällt/Wasser = 1:4 (v/v) suspendiert (es wurde ein pH-Wert von pH=4 gemessen). Anschließend wurden 6,05 kg einer 9,09 % igen wässrigen Natriumphosphat-Lösung zu getropft und der pH-Wert auf pH=7,2 eingestellt. Es wurde ca. 50 Minuten bei 23°C nachgerührt (pH=7,17). Anschließend wurden 0,65 kg einer 9,09 %igen wässrigen Natriumphosphat-Lösung zu getropft und der pH-Wert auf pH=7,5 eingestellt. Es wurde innerhalb einer Stunde auf 50°C Inntemperatur erwärmt und 3,0 Stunden bei dieser Temperatur nach gerührt. Es wurde innerhalb einer Stunde auf 22°C abgekühlt und übers Wochenende (ca. 64 Stunden*) bei dieser Temperatur nachgerührt. Das Kristallisat wird über eine K800 Seitzfilterplatte abfiltriert und einmal mit 1,6 L und einmal mit 0,8 L eines Gemisches bestehend aus Ethanol, Toluol vergällt/Wasser = 20:80 (v/v) und zweimal mit je 1,6 L Wasser nachgewaschen. Feuchtausbeute: 1,82 kg. Das Feuchtprodukt wurde anschließend bei 50°C unter Vakuum (< 100 mbar) bis zur Massen Konstanz getrocknet (ca. nach 17 Stunden Massen Konstant). Ausbeute: 0,978 kg (97,8 % d. Th.) eines farblosen kristallinen Pulvers.

*) aus technischen Gründen wurde übers Wochenende nachgerührt, in der Regel reichen 14 Stunden Nachrührzeit aus.

### Beispiel 3c

### Herstellung Reinprodukt (I)

250 g des in Beispiel 3b hergestellten Rohproduktes (I) wurden in 5000 ml Ethanol, Toluol vergällt suspendiert und anschließend wurde zum Rückfluss erhitzt. Das Produkt ging dabei in Lösung. Es wurde eine Stunde bei dieser Temperatur nachgerührt. Die Lösung wurde über einen beheizten Druckfilter (T=85°C) abfiltriert, der Druckfilter anschließend mit 200 ml Ethanol, Toluol vergällt nachgespült. Es wurde anschließend das Lösungsmittel soweit ab destilliert (ca. 4200 ml wurden ab destilliert) bis ein Endvolumen von ca. 4 fach (bzg. auf eingesetzte Substanz: 250 g x 4 ∼ 1000 ml) erreicht wurde. Anschließend wurde auf 4-5°C Innentemperatur abgekühlt (Rampe: Dauer ca. 4 Stunden). Dann wurde eine Stunde bei 4-5 °C Innentemperatur nach gerührt. Das Produkt wurden abfiltriert und einmal mit 220 ml Ethanol, Toluol vergällt nachgewaschen. Feuchtausbeute: 251,2 g. Das Feuchtprodukt wurde bei 50°C übers Wochenende (> 48 h) unter Vakuum (< 100 mbar) getrocknet. Ausbeute: 223,0 g (89,2 % d.Th.) eines farblosen kristallinen Pulvers.

Die nachfolgende Tabelle 4 fasst die Ergebnisse von 3 Kampagnen ausgehend von jeweils 2 kg Racemat von Finerenone (II) zusammen:

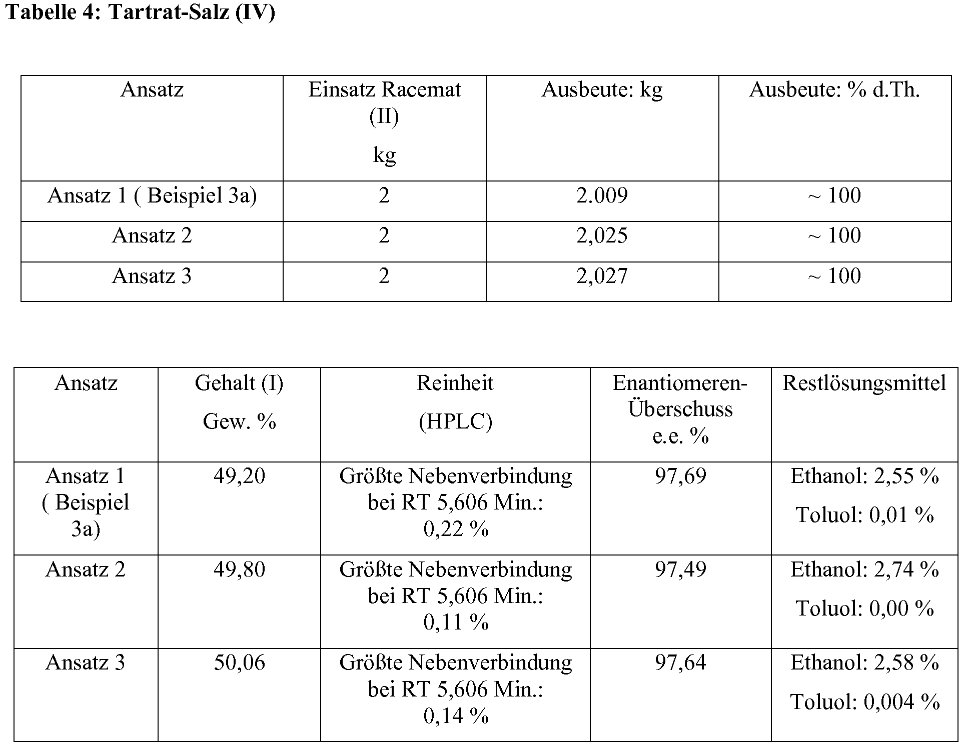

### Roh-Produkt (I)

| Ansatz | Einsatz Tartrat-Salz (IV): kg | Ausbeute: kg | Ausbeute: % d.Th. |
|---|---|---|---|
| Ansatz 1 (Beispiel 3b) | 2.009 | 0,978 | 97,8 |
| Ansatz 2 | 2,025 | 0,985 | 98,5 |
| Ansatz 3 | 2,027 | 0,987 | 98,7 |

| Ansatz | Gehalt (I) Gew. % | Reinheit (HPLC) | Enantiomeren-Überschuss e.e. % | Restlösungsmittel |
|---|---|---|---|---|
| Ansatz 1 | 98,10 | Finerenone (I): 99,85 % | 98,15 | Ethanol: 0,203 % |
| ( Beispiel 3a) | | Größte Nebenverbindung Verunreinigung E: 0,07 % | | Toluol: 0,117 % |
| Ansatz 2 | 99,00 | Finerenone (I): 99,83 % | 97,93 | Ethanol: 0,110 % |
| | | Größte Nebenverbindung Verunreinigung E: 0,08 % | | Toluol: 0,073 % |
| Ansatz 3 | 99,70 | Finerenone (I): 99,84 % | 97,97 | Ethanol: 0,118 % |
| | | Größte Nebenverbindung Verunreinigung E: 0,07 % | | Toluol: 0,076 % |

| | | | | |
|---|---|---|---|---|
| In allen 3 Ansätzen war der (+)-O,O-Dibenzoyl-D-Weinsäure-Gehalt: 0,00% | | | | |

### Rein-Produkt (I)

| Ansatz | Rohprodukt (I) g | Ausbeute: g | Ausbeute: % d.Th. |
|---|---|---|---|
| Ansatz 1 (Beispiel 3c) | 250 | 223 | 89,2 |
| Ansatz 2 | 250 | 235 | 94,0 |
| Ansatz 3 | 250 | 226 | 91,8 |

| Ansatz | Gehalt (I) Gew. % | Reinheit (HPLC) | Enantiomeren-Überschuss e.e.% | Restlösungsmittel |
|---|---|---|---|---|
| Ansatz 1 (Beispiel 3a) | 99,40 | Finerenone (I): | 100,00 | Ethanol: 0,074 % |
| | | 99,82 % Verunreinigung E 0,08 % | | Toluol: 0,000 % |
| Ansatz 2 | 99,80 | Finerenone (I): 99,94 % | 100,00 | Ethanol: 0,037 % Toluol: 0,000 % |
| | | Verunreinigung E 0,08 % | | |
| Ansatz 3 | 99,50 | Finerenone (I): 99,85 % | 99,92 | Ethanol: 0,033 % Toluol: 0,000 % |
| | | Verunreinigung E 0,08 % | | |

| | | | | |
|---|---|---|---|---|
| In allen 3 Ansätzen war der (+)-O,O-Dibenzoyl-D-Weinsäure-Gehalt (III) 0,00% | | | | |

### Beispiel 4

Betriebliche Umsetzung des Verfahrens. Die nachfolgenden Beispiele beschreiben die Umsetzung des Verfahrens im betrieblichen Maßstab. Die Ergebnisse, wie Ausbeute und Analytik von 2 Batchen Finerenone, rein (I) werden im Anschluss tabellarisch dargestellt:

### Beispiel 4a

### Tartrat-Salz (IV) Herstellung

Die Ansatzgröße entsprach 80 kg racemisches Finerenone (II).
- Im einem1600 L Rührwerk wurde bei IT 20°C 711,0 kg Branntwein vorgelegt, anschließend kamen 300 kg Wasser, dann 80,0 kg Racemat (II) und abschließend 41,7 kg (+)-Dibenzoyl-Weinsäure (III) hinzu.
- Es wurde auf IT 75°C erwärmt und 3h nachgerührt.
- Es wurde über 5h (Rampe) auf IT 23°C abgekühlt und 16h nachgerührt
- Die Suspension wurde zum Zentrifugieren auf die Stülpzentrifuge überführt, abgeschleudert und mit einem Gemisch, bestehend aus 10,7 kg Branntwein und 4,5 kg Wasser nachgewaschen, anschließend trocken geschleudert und ausgebracht.
- Die Trocknung erfolgte in einem 300 1 Kugeltrockner bei einer Manteltemperatur von 50°C und 30 mbar. Der Abbruch der Trocknung erfolgte bei einer Innentemperatur von 45°C (Dauer ca. 6h).
- Man kühlte auf 15°C und trug das Produkt anschließend aus.

### Beispiel 4b

### Rohprodukt (I)

Die Ansatzgröße entsprach 89,3 kg Tartrat-Salz (IV) zum Einsatz
- Herstellung einer Natriumphosphat-Lösung
- In einem 630 L Rührwerk wurde bei 20°C 360,0 kg VE-Wasser vorgelegt
- 36,0 kg Natriumphosphat wurde zugegeben und gelöst
- In einem 1600 L Rührwerk wurde bei 20°C 141,1 kg Branntwein vorgelegt
- Dann wurden 714,2 kg Wasser und 89,3 kg Tartrat-Salz (IV) zugegeben
- Es wurde auf eine Innentemperatur von 50°C erwärmt
- Dann wurde 295,1 kg einer Natriumphosphat-Lösung zu dosiert
- Nach 1,5 h wurde der pH-Wert durch Zugabe von 35,4 kg Natriumphosphat-Lösung auf pH=7,5 (+/-0,1) eingestellt
- Man ließ Nachrühren und kühlte über eine Rampe (3h) auf IT=22°C ab.
- Anschließend wurde bei IT=22°C nachgerührt (18h)
- In einer Vorlage wurde ein Gemisch aus 129,6 kg Wasser und 25,6 kg Branntwein hergestellt (zum Nachwaschen)
- Die Suspension wurde auf die Stülpzentrifuge auf dosiert (ca. 4 Chargen + Rest Charge) und portionsweise einmal mit dem Gemisch aus 129,6 kg Wasser und 25,6 kg Branntwein und einmal mit 288,0 kg VE-Wasser gewaschen.
- Das Produkt wurde ausgetragen und im Kugeltrockner getrocknet (TM=50°C, 30 mbar, Abbruch IT=45°C, ca. 6h)
- Man kühlte ab und trug das Produkt bei <30°C aus

### Beispiel 4c

### Umarbeitung des Rohproduktes (I)

In manchen Fällen, wenn der Spezifikationsgrenzwert für (+)-Dibenzoyl-Weinsäure (III) im Rohprodukt > 0,1 % ist, hat es sich als vorteilhaft erwiesen, eine Umarbeitung durch zuführen (siehe Beispiel 6), die garantiert, dass der Grenzwert < 0,1 % reproduzierbar erreicht wird. Das Umarbeitungs-Verfahren ist zudem nahezu verlustfrei und liefert das Rohprodukt in der Regel in > 95% Ausbeute.

### Umgearbeitetes Rohprodukt (I)

Die Ansatzgröße entsprach 30 kg Rohprodukt (I)
- Herstellung einer Natriumphosphat-Lösung: 2,3 kg Natriumphosphat wurden bei Raumtemperatur in 147,8 kg Wasser in einer 250 L Rührvorlage gelöst
- Bei 20°C wurden in einem 630 L Rührwerk 198,8 kg Branntwein vorgelegt und 120 kg Wasser zugegeben, anschließend wurden 30 kg Rohprodukt (enthaltend (+)-Dibenzoyl-Weinsäure (III) wie in der Tabelle 5 angegeben zugesetzt.
- Es wurde auf 70°C Innentemperatur erwärmt und 30 Minuten nachgerührt.
- Bei 70°C wurden 14,1 kg der oben hergestellten Natriumphosphat-Lösung zu dosiert und ein pH-Wert von pH 8,9 (+/- 0,1) und 20 Stunden bei 70°C nachgerührt.
- Über eine Rampe wurde innerhalb 60 Minuten auf 44°C abgekühlt und 300 g Impfkristalle Finerenone, rein (I) zugegeben
- Anschließend wurde bei 200 mbar und einer maximalen Inntemperatur von 60°C ab destilliert und parallel 369 kg Wasser ins Rührwerk zu dosiert.
- Zum Schluss wurde innerhalb 1,5 Stunden auf 22°C runter gekühlt und das Produkt auf einer Drucknutsche isoliert und in 3 Portionen mit insgesamt 180 L Wasser gewaschen
- Anschließend wurde bei 50°C Manteltemperatur unter Vakuum (30 mbar) bis zur Gewichtskonstanz getrocknet.
- Man ließ auf 15°C abkühlen und trug das Produkt aus

### Beispiel 4d

### Reinprodukt (I)

Die Ansatzgröße entsprach 26 kg (2 mal 13 kg) umgearbeitetes Rohprodukt (I)
- In einem 250 L Rührwerk wurden 13 kg umgearbeitetes Rohprodukt (I) in 184,9 kg Branntwein bei 20°C vorgelegt und das Rührwerk zuvor inertisiert
- Die Lösung wurde auf 80°C Manteltemperatur erhitzt und 30 Minuten bei 78°C nachgerührt (dabei geht das Produkt in Lösung).
- Die Lösung wurde über eine 0,65 um Filterkerze (PP-Kerze Sartorius) filtriert (GMP-Filtration) und das Filtrat in ein 630L Rührwerk überführt.
- Es wurde mit 18,5 kg Branntwein nachgewaschen
- Dieses wurde in 2 Teilportionen (2 mal 13 kg Produkt) wiederholt und die Lösungen vereinigt
- Es wurde zu einem finalen Füllstand von ca. 130 L bei Normaldruck abdestillert (in zwei Portionen, Manteltemperatur: 104°C, Dauer ca. 6 Stunden)
- Die Lösung wurde über eine lineare Temperaturrampe über 4 Stunden auf 0°C runtergekühlt und anschließend wurde eine Stunde nach gerührt.
- Die Produkt-Suspension wurde über eine Drucknutsche isoliert und mit 45,9 kg Branntwein gewaschen
- Das Feuchtprodukt wurde anschließend unter Vakuum (30 mbar) bei 50°C bis zur Gewichtkonstanz (ca. 12 h) getrocknet. Es wurde auf 15°C abgekühlt und das Produkt ausgebracht.

Übersicht der Ausbeute und analytischen Ergebnisse von 3 Betriebs-Ansätzen zur Herstellung von reinem Finerenone (I) ist in Tabelle 5 dargestellt

### Tartrat-Salz (IV)

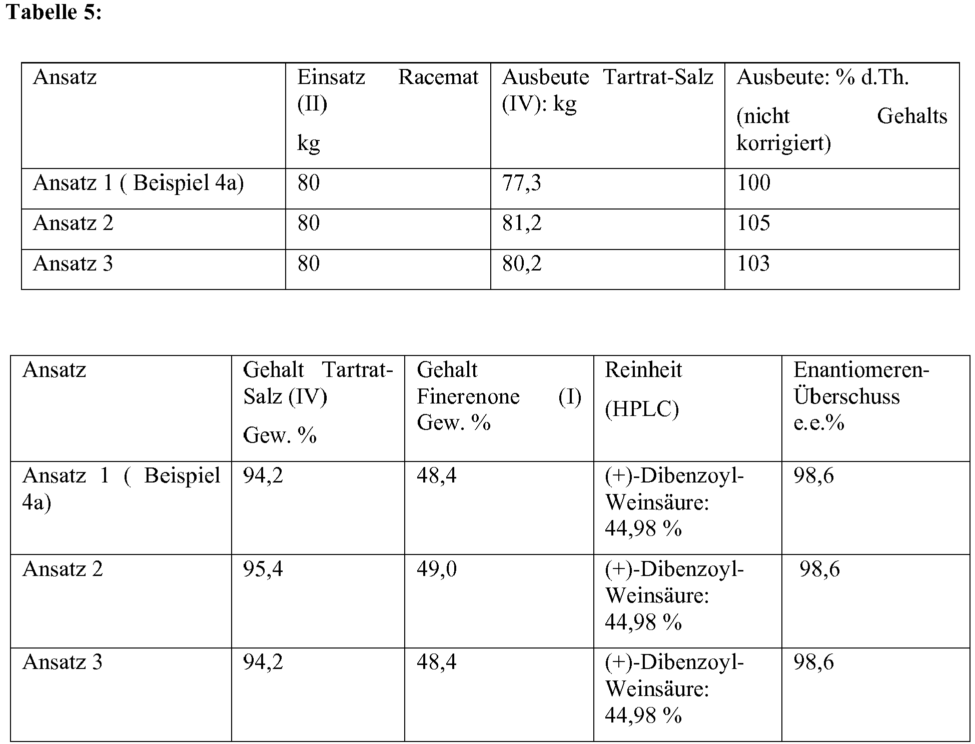

### Roh-Produkt (I)

| Ansatz | Einsatz Tartrat-Salz (IV) kg | Ausbeute (I, roh): kg | Ausbeute: % d.Th. |
|---|---|---|---|
| Ansatz 1 (Beispiel 4b) | 89,3 | 36,6 | 80 |
| Ansatz 2 | 89,3 | 44,2 | 96 |
| Ansatz 3 | 89,3 | 45,7 | 99 |

| Ansatz | Gehalt (I) Gew. % | Reinheit (HPLC) | Enantiomeren-Überschuss e.e.% | Restlösungsmittel |
|---|---|---|---|---|
| Ansatz 1 ( Beispiel 4b) | 98,5 | Finerenone (I): | 98,8 | Ethanol: 0,098 % |
| | | 99,71 % | | Toluol: 0,048 % |
| | | Verunreinigung E: 0,08% | | |
| | | Summe aller organischer Neben-Komponenten: 0,29% | | |
| | | (+)-Dibenzoyl-Weinsäure: 0,71 % | | |
| Ansatz 2 | 98,2 | Finerenone (I): 97,70 % | 98,9 | Ethanol: 0,151 % |
| | | Verunreinigung E: 0,08% | | Toluol: 0,088 % |
| | | Summe aller organischer Neben-Komponenten: 0,30 % | | |
| | | (+)-Dibenzoyl-Weinsäure: 0,39 % | | |

| | | | | |
|---|---|---|---|---|
| Ansatz 3 | 100 | Finerenone (I): | 98,7 | Ethanol: 0,102 % |
| | | 99,76 % Verunreinigung E: 0,07% | | Toluol: 0,066 % |
| | | Summe aller organischer Neben-Komponenten: 0.0,24 % | | |
| | | (+)-Dibenzoyl-Weinsäure: 0,46% | | |

### Roh-Produkt (I) nach Umarbeitung

| Ansatz | Einsatz (I): kg | Ausbeute (I): kg | Ausbeute: % d.Th. |
|---|---|---|---|
| Ansatz 1 (Beispiel 4c) | 30 | 29,8 | 99,33 |
| Ansatz 2 | 30 | 29,8 | 99,33 |

| Ansatz | Gehalt (I) Gew. % | Reinheit (HPLC) | Enantiomeren-Überschuss e.e.% | Restlösungsmittel |
|---|---|---|---|---|
| Ansatz 1 ( Beispiel 4c) | 99,6 | Finerenone (I): | 98,8 | Ethanol: n.n. |
| | | 99,70 % | | Toluol: n.n. |
| | | **(+)-Dibenzoyl-Weinsäure: < 0,05 %** | | |
| | | Größste Nebenkomponente Verunreinigung E: 0,08 % | | |
| | | Summe aller organischer Neben-Komponenten: 0.3 % | | |
| Ansatz 2 | 99,6 | Finerenone (I): | 98,8 | Ethanol: n.n. |
| | | 99,70 % | | Toluol: n.n. |
| | | **(+)-Dibenzoyl-Weinsäure: < 0,05 %** | | |
| | | Größste Nebenkomponente Verunreinigung E: 0,08 % | | |
| | | Summe aller organischer Neben-Komponenten: 0.3 % | | |

### Rein-Produkt, Finerenone, rein (I)

| Ansatz | Einsatz kg | Ausbeute: kg | Ausbeute: % d.Th. |
|---|---|---|---|
| Ansatz 1 (Beispiel 4d) | 26 | 23,1 | 88,85 |
| Ansatz 2 | 26 | 22,9 | 88,08 |

| Ansatz | Gehalt (I) Gew. % | Reinheit (HPLC) | Enantiomeren-Überschuss e.e.% | Restlösungsmittel |
|---|---|---|---|---|
| Ansatz 1 ( Beispiel 4d) | 100 | Finerenone (I): | 100 | Ethanol: 0,044 |
| | | 99,8 % | | Toluol: 0,00 |
| | | Summe aller organischer Neben-Komponenten: 0.2 % | | Wasser: < 0,2 |
| | | Größte Nebenkomponente Verunreinigung E: 0,08 % | | |
| | | (+)-Dibenzoyl-Weinsäure: < 0,05 | | |
| Ansatz 2 | 100 | Finerenone (I): | 100 | Ethanol: 0,063 |
| | | 99,8 % | | Toluol: 0,00 |
| | | Summe aller organischer Neben-Komponenten: 0.2 % | | Wasser: < 0,2 |
| | | Größte Nebenkomponente Verunreinigung E: 0,08 % | | |
| | | (+)-Dibenzoyl-Weinsäure: < 0,05% | | |

### Beispiel 5

Zur Freisetzung des rohen Finerenone (I) eignen sich mehrere Varianten analog der Vorschrift aus Beispiel 1b. Ziel dabei war es den Anteil an (+)-O,O-Dibenzoyl-D-Weinsäure (III) im Rohprodukt möglichst klein zu halten (0,15 % und weniger). Dabei wurden im Einzelnen End-pH-Wert nach Zudosierung der Natriumphosphat-Lösung, Temperatur, Dosierzeit der Natriumphosphat-Lösung und Nachrührzeit variiert und der Einfluss auf den Gehalt von (+)-O,O-Dibenzoyl-D-Weinsäure im Rohprodukt untersucht.

Ansatzgröße: 100 g Tartrat-Salz (IV) in 158,0 g Branntwein und 799,8 g Wasser
Analytik-Daten zu eingesetztem Tartrat-Salz (IV):

| Finerenone (I) | 48,05 % Gewichtsprozent (HPLC) |
|---|---|
| Enantiomerenüberschuss | 97,71 % e.e. |
| Größte unbekannte Nebenkomponente bei Rt 5,606 Min. | 0,28 % |
| Restlösungsmittel: | |
| EtOH | 2,576 % |
| Toluol | 0,006 % |

Die nachfolgende Tabelle 6 fasst die Ergebnisse zusammen:

**Tabelle 6**

| **Ansatz** | **pH-Wert** | **Menge Na₃PO₄**-**Lsg. (g) (100g/1L Wasser)** | **T (°C)** | **Dosierzeit (Min.)** | **Nachrührzeit (Min.)** | **Reinheit Finerenone (I) HPLC (%)** | **Ausbeute (% d.Th.)** | **Anteil an (+)-Di-Benzoyl-Weinsäure (Gew. %)** |
|---|---|---|---|---|---|---|---|---|
| 1 | 7,5 | 400 | 50 | 30 | 180 | 99,17 | 97,2 | 0,0996 |
| 2 | 7,5 | 400 | 60 | 30 | 180 | 99,74 | 97,2 | 0,1101 |
| 3 | 8,0 | 444,9 | 50 | 30 | 180 | 99,86 | 97,0 | 0,0986 |
| 4 | 8,5 | 467,7 | 50 | 30 | 180 | 99,73 | 96,6 | 0,1298 |
| 5 | 9,0 | 480,3 | 50 | 30 | 180 | 99,85 | 97,4 | 0,1027 |
| 6 | 7,5 | 400 | 50 | 30 | 60 | 99,86 | 96,8 | 0,1010 |
| 7 | 7,5 | 400 | 50 | 30 | 300 | 99,87 | 97,2 | 0,1058 |
| 8 | 7,5 | 400 | 50 | 8 | 180 | 99,73 | 97,6 | 0,1294 |

### Beispiel 6

### Umarbeitungs-Verfahren

### Beispiel 6a

### Umgearbeites Rohprodukt: Reduzierung des Anteils (+)-O,O-Dibenzoyl-D-Weinsäure < 0,1 %

Dieses Experiment diente dazu die Robustheit des Umarbeitungs-Verfahrens zu demonstrieren, indem bewusst eine Charge mit erhöhtem (+)-O,O-Dibenzoyl-D-Weinsäure (III) Anteil eingesetzt wurde.

Ein Rohprodukt das laut Analytik noch 0,77 % (+)-O,O-Dibenzoyl-D-Weinsäure (III) enthielt wurde zur Umarbeitung eingesetzt

| Finerenone, roh (I) | Reinheit: 99,67% Fläche (HPLC); Gehalt: 98,69 Gew. % |
|---|---|
| (+)-O,O-Dibenzoyl-D-Weinsäure | 0,77 % |
| Größte Nebenkomponente Verunreinigung E | 0,07 % |

### Umarbeitung des Rohproduktes: Reduzierung des Anteils (+)-O,O-Dibenzoyl-D-Weinsäure (III) < 0,1 %

Ein Rohprodukt das laut Analytik noch 0,77 % (+)-O,O-Dibenzoyl-D-Weinsäure (III) enthält wurde zur Umarbeitung eingesetzt. Hierzu wurde wie folgt vorgegangen:
150 g Rohprodukt (I) (0,77% (+)-O,O-Dibenzoyl-D-Weinsäure (III) enthaltend) wurden in 600 g Wasser und 953,9 g Branntwein gegeben und auf 70°C erhitzt. Innerhalb einer Stunde ging alles in Lösung. Anschließend wird 30 Minuten bei 70°C nachgerührt. Der pH-Wert beträgt pH=5,0. Der pH-Wert wurde durch Zugabe von 44,6 g einer wässrigen Natriumphosphat-Lösung (15 g Natriumphosphat auf 985 g Wasser) auf pH=8,5 gestellt und anschließend 2 Stunden bei 70°C gerührt. Innerhalb von 30 Minuten wurde auf 53°C abgekühlt und mit reinem Material (Impfkristalle) an geimpft. Dann wurde 30 Minuten bei 50-52°C nachgerührt. Bei 200 bis 155 mbar und einer Innentemperatur wurde das Ethanol nahezu vollständig ab destilliert und parallel 1845 ml Wasser zu dosiert (der Füllstand wurde konstant gehalten: ab destillierte Menge = zugegebene Menge Wasser). Die Heizung wurde abgeschaltet und die Suspension über Nacht bei 23°C nachgerührt. Das Kristallisat wurde über eine Fritte abfiltriert und dreimal mit je 300 ml Wasser gewaschen. Feuchtausbeute: 218,9 g. Das Feuchtprodukt wurde bei 50°C übers Wochenende (> 48 Stunden) im Vakuum (< 100 mbar) getrocknet. Ausbeute: 146,7 g (97,8 % d. Th.)

| Finerenone (I) | Reinheit: 99,87 Fläche (HPLC); Gehalt: 100,3 Gew. % |
|---|---|
| Enantiomerenüberschuss | 97,87 % e.e. |
| (+)-O,O-Dibenzoyl-D-Weinsäure | 0,00 % |
| Größte Nebenkomponente Verunreinigung E | 0,07 % |
| Restlösungsmittel: | |
| EtOH | 0,286 % |

### Beispiel 6b

### Demonstration der Stabilität, Rühren 20 h bei 70°C

### Umarbeitung des Rohproduktes: Reduzierung des Anteils (+)-O,O-Dibenzoyl-D-Weinsäure (III) ≤ 0,1 %

Ein Rohprodukt das laut Analytik noch 0,77 % (+)-O,O-Dibenzoyl-D-Weinsäure (III) enthält wurde zur Umarbeitung eingesetzt. Hierzu wurde wie folgt vorgegangen:
150 g Rohprodukt (I) (0,77% (+)-O,O-Dibenzoyl-D-Weinsäure (III) enthaltend) wurden in 600 g Wasser und 953,9 g Branntwein gegeben und auf 70°C erhitzt. Innerhalb einer Stunde ging alles in Lösung. Anschließend wird 30 Minuten bei 70°C nachgerührt. Der pH-Wert beträgt pH=5,0. Der pH-Wert wurde durch Zugabe von 45,2 g einer wässrigen Natriumphosphat-Lösung (15 g Natriumphosphat auf 985 g Wasser) auf pH=8,5 gestellt und anschließend 20 Stunden bei 70°C gerührt. Innerhalb von 30 Minuten wurde auf 53°C abgekühlt und mit reinem Material (Impfkristalle) an geimpft. Dann wurde 30 Minuten bei 50-52°C nachgerührt. Bei 200 bis 155 mbar und einer Innentemperatur wurde das Ethanol nahezu vollständig ab destilliert und parallel 1845 ml Wasser zu dosiert (der Füllstand wurde konstant gehalten: ab destillierte Menge = zugegebene Menge Wasser). Die Heizung wurde abgeschaltet und die Suspension über Nacht bei 23°C nachgerührt. Das Kristallisat wurde über eine Fritte abfiltriert und dreimal mit je 300 ml Wasser gewaschen. Feuchtausbeute: 194,3 g. Das Feuchtprodukt wurde bei 50°C über Nacht (ca. 16 Stunden) im Vakuum (< 100 mbar) getrocknet. Ausbeute: 143,1 g (95,4 % d. Th.)

| Finerenone (I) | Reinheit: 99,86 Fläche (HPLC); Gehalt: 101,3 Gew. % |
|---|---|
| Enantiomerenüberschuss | 97,85 % e.e. |
| (+)-O,O-Dibenzoyl-D-Weinsäure | 0,00 % Fläche (0,024 Gew. %) |
| Größte Nebenkomponente Verunreinigung E | 0,07 % |
| Restlösungsmittel: | |
| EtOH | 0,128 % |

### Beispiel 6 c

### Umarbeitung des Rohproduktes: Reduzierung des Anteils (+)-O,O-Dibenzoyl-D-Weinsäure (III) < 0,1 %. Rücktitration des pH-Wertes auf pH 7,0 mit Natriumdihydrogenphosphat

Ein Rohprodukt das laut Analytik noch 0,77 % (+)-O,O-Dibenzoyl-D-Weinsäure (III) enthält wurde zur Umarbeitung eingesetzt. Hierzu wurde wie folgt vorgegangen:
375,0 g Rohprodukt (I) (0,77% (+)-O,O-Dibenzoyl-D-Weinsäure (III) enthaltend) wurden in 1500 g Wasser und 2384,8 g Branntwein gegeben und auf 70°C erhitzt. Innerhalb einer Stunde ging alles in Lösung. Anschließend wird 30 Minuten bei 70°C nachgerührt. Der pH-Wert beträgt pH=4,9. Der pH-Wert wurde durch Zugabe von 101,4 g einer wässrigen Natriumphosphat-Lösung (15 g Natriumphosphat auf 985 g Wasser) auf pH=8,5 gestellt und anschließend 20 Stunden bei 70°C gerührt. Anschließend wurde der pH-Wert mit einer Natriumdihydrogenphosphat-Lösung (50 g Natriumdihydrogenphosphat 50 g in 200 ml Wasser) auf pH=7 eingestellt. Innerhalb von 30 Minuten wurde auf 53°C abgekühlt und mit reinem Material (Impfkristalle) an geimpft. Dann wurde 30 Minuten bei 50-52°C nachgerührt. Bei 200 bis 155 mbar und einer Innentemperatur wurde das Ethanol nahezu vollständig ab destilliert und parallel 4610 ml Wasser zu dosiert (der Füllstand wurde konstant gehalten: ab destillierte Menge = zugegebene Menge Wasser). Anschließend wurde innerhalb 90 Minuten auf 23°C abgekühlt. Dann wurde übers Wochenende (ca. 70 h) bei 22°C nachgerührt. Das Kristallisat wurde über eine Fritte abfiltriert und dreimal mit je 750 ml Wasser gewaschen. Feuchtausbeute: 402,3 g. Das Feuchtprodukt wurde bei 50°C über Nacht (ca. 16 Stunden) im Vakuum (< 100 mbar) getrocknet. Ausbeute: 336,6 g (89,76 % d. Th.)

| Finerenone, roh (I) | Reinheit: 99,84 Fläche (HPLC); Gehalt: 99,2 Gew. % |
|---|---|
| Enantiomerenüberschuss | 98,02 % e.e. |
| (+)-O,O-Dibenzoyl-D-Weinsäure | 0,00 % Fläche (0,0068 Gew. %) |
| Größte Nebenkomponente Verunreinigung E | 0,07 % |
| Restlösungsmittel: | |
| EtOH | 0,107 % |
| Toluol | 0,001 % |

### Beispiel 6d

### Finale Kristallisation des Batches aus Beispiel 6c

80,0 g des in Beispiel 6c hergestellten Rohproduktes (I) wurden in 1600 ml Ethanol, Toluol vergällt suspendiert und anschließend wurde zum Rückfluss erhitzt. Das Produkt ging dabei in Lösung. Es wurde eine Stunde bei dieser Temperatur nachgerührt. Die Lösung wurde über einen beheizten Druckfilter (T=75°C) abfiltriert, anschließend mit 100 ml Ethanol, Toluol vergällt nachgespült. Es wurde anschließend das Lösungsmittel unter Vakuum (Mantel-temperatur 45-47°C) soweit ab schonend abdestilliert bis ein Endvolumen von ca. 5 fach (bzg. auf eingesetzte Substanz: 80g x 5 ∼ 400 ml) erreicht wurde. Dann wurde innerhalb einer Stunde auf 2°C Innentemperatur abgekühlt und ca. 1 h bei dieser Temperatur gerührt. Das Produkt wurde abfiltriert und einmal mit 80 ml Ethanol, Toluol vergällt nachgewaschen. Feuchtausbeute: 83,3 g. Das Feuchtprodukt wurde bei 50°C über Nacht ca. 16 h) unter Vakuum (< 100 mbar) getrocknet. Ausbeute: 71,4 g (89,3 % d.Th.) eines farblosen kristallinen Pulvers, feine Nadelkristalle.

### Analytische Ergebnisse:

| Finerenone (I) | Reinheit: 99,82 Fläche (HPLC); Gehalt: 98,7 Gew. % |
|---|---|
| Enantiomerenüberschuss | 99,87 % e.e. |
| Größte Nebenkomponente Verunreinigung E | 0,08 % |
| Restlösungsmittel: | |
| EtOH | 0,489 % |
| Toluol | 0,005 % |

### Beispiel 7

### Isolierung des Finerenone-Enantiomers (Ia)

### Beispiel 7a

### Herstellung des (-)-O,O-Dibenzoyl-L-Weinsäure Tartrat-Salzes

187,2 g Racemat (II) wurden bei Raumtemperatur vorgelegt und anschließend 1662,5 g Branntwein zu gegeben. Dann wurde 485,5 g Wasser zugesetzt. Es wurden 97,5 g % (-)-O,O-Dibenzoyl-L-Weinsäure (IIIa) über einen Feststofftrichter zugegeben und anschließend mit 156,0 g Wasser nachgespült. Die Suspension wurde innerhalb einer Stunde auf 75°C Innentemperatur erwärmt und anschließend 3 Stunden bei 75°C gerührt. Anschließend wurde innerhalb von 5,0 Stunden über eine Abkühlrampe ab 23°C abgekühlt und über Nacht (ca. 18 Stunden) bei dieser Temperatur nachgerührt. Die Suspension wurde über eine Fritte abfiltriert und zweimal mit einem Gemisch bestehend aus 178 g Branntwein und 75 g Wasser nachgewaschen nach gewaschen. Feuchtausbeute: 255,4 g. Das Feuchtprodukt wurde anschließend über Nacht (ca. 16 Stunden) bei 50°C unter Vakuum (< 100 mbar) getrocknet. Ausbeute: 188,6 g (100,73 % d.Th.) eines farblosen kristallinen Pulvers. Die Mutterlauge und Waschlösung wurde vereinigt (ca. 3200 ml gelbliche Lösung, pH = 4,6) und hieraus das Finerenone, roh (I) isoliert (Beispiel 7b)

| Finerenone-Enantiomer (Ia) | 49,3 % Gewichtsprozent (HPLC) |
|---|---|
| Enantiomerenüberschuss | 97,35 % e.e. |
| Restlösungsmittel: | |
| EtOH | 1,847 % |
| Toluol | 0,00% |

In analoger Weise, wie in Beispiel 1b und 1c beschrieben kann aus diesem Tartrat-Salz das Enantiomer von Finerenone (Ia) hergestellt werden.

### Beispiel 7b

### Isolierung von Finerenone, roh (I) aus der Mutterlauge

Die vereinigte Mutterlauge und Waschlösung aus Beispiel 7a (ca. 3200 ml gelbliche Lösung, pH = 4,6) wurde bei Raumtemperatur durch Zugabe von 43,3 g einer wässrigen Natriumphosphat-Lösung (100 g in 1 L Wasser gelöst) auf pH = 7,6 eingestellt. Anschließend wurd unter reduziertem Druck (85 bis 65 mbar, 38 bis 20°C Innentemperatur) der Branntwein weitgehend ab destilliert und auf ein Endvolumen von ca. 0,8 L reduziert. Es wurde auf Raumtemperatur abgekühlt und die ausgefallene Suspension 2 Stunden bei 22°C gerührt. Die Suspension wurde abfiltriert und 2-mal mit je 150 ml Wasser gewaschen. Feuchtausbeute: 159,1 g. Das Feuchtprodukt wurde bei 50°C über Nacht (ca. 16 h) unter Vakuum (< 100 mbar) getrocknet. Ausbeute: 86,3 g (92,2 % d. Th. bezogen auf in Beispiel 7a eingesetztes Racemat (II)).

| Finerenone (I) | Reinheit: 99,48 % Fläche (HPLC); Gehalt: 99,39 Gew. % |
|---|---|
| Enantiomerenüberschuss | 98,14 % e.e. |
| Größte Nebenkomponente Verunreinigung E | 0,13 % |
| (-)-O,O-Dibenzoyl-L-Weinsäure | 0,14 % (0,05 Gew. %) |

In analoger Weise, wie in Beispiel 1c beschrieben, kann aus diesem Rohprodukt Finerenone (I) in reiner Form hergestellt werden.

### Beispiel 8

### Beispiel 8a

### Isolierung vom Fehlenantiomer (Ia) aus der Mutterlauge

Die vereinigte Mutterlauge und Waschlösung aus Beispiel 1a (ca. 3750 ml gelbliche Lösung, pH = 4,5) wurde bei Raumtemperatur durch Zugabe von 101,1 g einer wässrigen Natriumphosphat-Lösung (100 g in 1 L Wasser gelöst) auf pH = 7,5 eingestellt. Anschließend wurde unter reduziertem Druck (85 bis 65 mbar, 38 bis 20°C Innentemperatur) der Branntwein weitgehend ab destilliert und auf ein Endvolumen von ca. 0,85 L reduziert. Es wurde auf Raumtemperatur abgekühlt und die ausgefallene Suspension, übers Wochenende gerührt (> 48 Stunden), anschließend weitere 2 Stunden bei 22°C gerührt. Die Suspension wurde abfiltriert und 2-mal mit je 200 ml Wasser gewaschen. Feuchtausbeute: 139,1 g. Das Feuchtprodukt wurde bei 50°C über Nacht (ca. 16 h) unter Vakuum (< 100 mbar) getrocknet. Ausbeute: 103,1 g (82,48 % d. Th. bezogen auf in Beispiel 1a eingesetztes Racemat (II)).

| Finerenone-Enantiomer (Ia) | Reinheit: 99,75 % Fläche (HPLC); Gehalt: 99,2 Gew. % |
|---|---|
| Enantiomerenüberschuss | 99,34 % e.e. |
| Größte Nebenkomponente Verunreinigung E | 0,12 % |
| (+)-O,O-Dibenzoyl-D-Weinsäure | 0,14 % (0,05 Gew. %) |
| Wasser | 0,102 % |

### Beispiel 8b

### Betriebsansatz zur Isolierung des Finerenone-Enantiomers (Ia)

Eine vereinigte Mutterlauge und Waschlösung aus Beispiel 4 (Tartrat-Herstellung im Betriebsmaßstab) wurde wie folgt aufgearbeitet: Menge ca. 1165 kg Lösung
- Herstellung einer Natriumphosphat-Lösung: 7,3 kg Natriumphosphat wurden in 73,1 kg Wasser bei 20°C gelöst
- In einem 1600 L Rührwerk wurden 1165 kg Mutterlauge (inclusive Waschlösung) bei 20°C vorgelegt und mit 28 kg der oben hergestellten Natriumphosphat-Lösung der pH auf 7,5 (+/-0,1) eingestellt
- Es wurde 0,25 h nachgerührt und anschließend unter Vakuum (65 mbar, IT ca. 22°C) Branntwein bis auf einen Füllstand von ca. 310 L abdestilliert
- Anschließend wurde 2 h bei 20°C nachgerührt und die Suspension über eine Stülpzentrifuge isoliert, wobei jeweils mit 20 kg Wasser gewaschen wurde.
- Das Feuchtprodukt wurde in einem Kugeltrockner bei 50°C unter Vakuum getrocknet (30 mbar; ca. 6 h)
- Es wurde auf 15°C abgekühlt und das Produkt ausgebracht

### Analytik-Daten der eingesetzten Mutterlauge (incl. Spül-Lösung) zu Ansatz 1

| | |
|---|---|
| Finerenone-Enantiomer (Ia) | 92,4 % (HPLC) |
| Enantiomerenüberschuss | 95,92 |
| Größte Nebenkomponente Verunreinigung E | 0,26 % |
| (+)-O,O-Dibenzoyl-D-Weinsäure | 5,38 % (HPLC) |

| Restlösungsmittel | |
|---|---|
| Ethanol | 0,319 % |
| Toluol | 0,001 % |

### Analytik-Daten der eingesetzten Mutterlauge (incl. Spül-Lösung) zu Ansatz 2

| | |
|---|---|
| Finerenone-Enantiomer (Ia) | 92,03 % |
| Enantiomerenüberschuss | 99,03 |
| Größte Nebenkomponente Verunreinigung E | 0,15 % |

| (+)-O,O-Dibenzoyl-D-Weinsäure | 5,94 % |
|---|---|
| | |
| Restlösungsmittel | |
| Ethanol | 0,177 % |
| Toluol | 0,001 % |

| Ansatz | Einsatz kg | Ausbeute: kg | Ausbeute: % d.Th. |
|---|---|---|---|
| Ansatz 1 | ∼ 1165 Mutterlauge (incl. Spül-Lösung) | 38,2 | 96 |
| Ansatz 2 | ∼ 1165 Mutterlauge (incl. Spül-Lösung) | 37,1 | 93 |

### Analytik-Daten Ansatz 1

| Finerenone-Enantiomer (Ia) | Reinheit: 99,71 % |
|---|---|
| Enantiomerenüberschuss | 96,56 % e.e. |
| Größte Nebenkomponente Verunreinigung E | 0,106 % |
| (+)-O,O-Dibenzoyl-D-Weinsäure | 0,081 % |

### Analytik-Daten Ansatz 2

| Finerenone-Enantiomer (Ia) | Reinheit: 99,446 % |
|---|---|
| Enantiomerenüberschuss | 99,5 % e.e. |
| Größte Nebenkomponente Verunreinigung E | 0,107 % |
| (+)-O,O-Dibenzoyl-D-Weinsäure | 0,33 % |

### Beispiel 9

Beispiele für weitere Weinsäure-Derivate und weitere Lösungsmittel

### Beispiel 9a

### (+)-O,O-Di-p-anisoyl-D-weinsäure

100 mg Racemat wurden mit 111 mg (+)-O,O-Di-p-anisoyl-D-weinsäure in 5 ml Dichlormethan gelöst und stehengelassen. Nach einiger Zeit fiel das Diastereomeren-Salz aus. Dieses wurde abfiltriert und der Enantiomeren-Überschuss gemessen. Die Messung ergab einen Enantiomeren-Überschuss von 68 % e.e. zugunsten von (Ia)

### Beispiel 9b

### (-)-O,O'-Di-p-toluyl-L-weinsäure

100 mg Racemat wurden mit 0,55 eq. (-)-O,O-Di-p-toluyl-L-weinsäure in 4 ml Lösungsmittel gelöst und stehengelassen. Nach einiger Zeit fiel das Diastereomeren-Salz aus. Dieses wurde abfiltriert und der Enantiomeren-Überschuss gemessen. Die Messung ergab einen Enantiomeren-Überschuss von y % e.e. zugunsten von (Ia). Nachfolgende Tabelle 7 fasst die Ergebnisse zusammen

**Tabelle 7:**

| **4 ml Lösungsmittel/Lösungsmittelgemisch** | **Enantiomeren-Überschuss e.e. % (Ia)** |
|---|---|
| n-Propanol / Wasser 80:20 | 85,35 |
| n-Pentanol / Wasser 90:10 | 89,10 |
| Methanol / Wasser 80:20 | 84,47 |
| Methanol | 93,18 |
| Isopropanol / Wasser 80:20 | 81,20 |
| Isobutanol / Wasser 90:10 | 79,25 |
| Ethylenglycol / Wasser 80:20 | 93,68 |
| Ethylenglycol | 68,50 |
| Ethanol / Wasser 80:20 | 85,87 |
| Ethanol | 85,96 |
| 2-Butanol / Wasser 80:20 | 83,92 |
| 1-Butanol / Wasser 90:10 | 88,93 |

### Beispiel 9c

### (+)-O,O'-Dibenzoyl-D-weinsäure

100 mg Racemat wurden mit 0,55 eq. (+)-O,O'-Dibenzoyl-D-weinsäure in 4 ml Lösungsmittel gelöst und stehengelassen. Nach einiger Zeit fiel das Diastereomeren-Salz aus. Dieses wurde abfiltriert und der Enantiomeren-Überschuss gemessen. Die Messung ergab einen Enantiomeren-Überschuss von y % e.e. zugunsten von (I). Nachfolgende Tabelle 8 fasst die Ergebnisse zusammen

**Tabelle 8:**

| **4 ml Lösungsmittel/Lösungsmittelgemisch** | **Enantiomeren-Überschuss e.e. % (I)** |
|---|---|
| n-Propanol / Wasser 80:20 | 94,01 |
| n-Pentanol / Wasser 90:10 | 94,59 |
| Methanol / Wasser 80:20 | 89,09 |
| Methanol | 91,21 |
| Isopropanol / Wasser 80:20 | 90,92 |
| Isobutanol / Wasser 90:10 | 85,42 |
| Ethylglycol | 92,08 |
| Ethanol / Wasser 80:20 | 94,64 |
| Ethanol | 93,64 |
| Cyclohexanol / Wasser 90:10 | 84,33 |
| Benzylalkohol / Wasser 90:10 | 88,83 |
| 2-Butanol / Wasser 80:20 | 92,49 |
| 1-Pentanol | 80,99 |
| 1-Butanol / Wasser 90:10 | 94,02 |

## Patentansprüche

1. Racemat-Spaltung von (II) 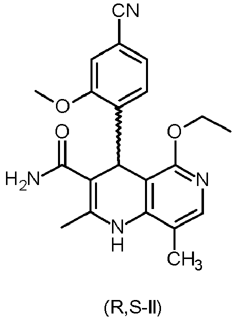 in (Ia) und/oder (I) 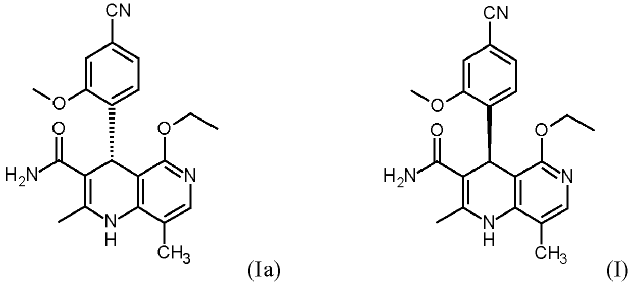 mit chiralen substituierten Weinsäureestern der allgemeinen Formeln (IIIa) oder (IIIb) 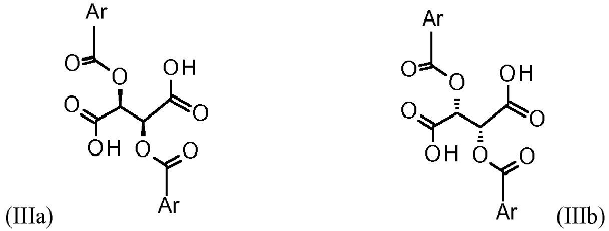 wobei Ar für einen unsubstituierten oder substituierten Aromaten oder Heteroaromaten steht.

2. Verfahren zur Herstellung von (4S)- 4-(4-Cyano-2-methoxyphenyl)-5-ethoxy-2,8-dimethyl-1,4-dihydro-1,6-naphthyridin-3-carbox-amid der Formel (I) 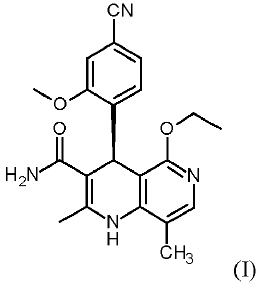 durch Racematspaltung von (II) 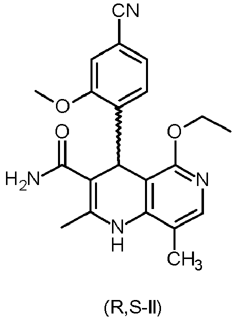 mit einem chiralen substituierten Weinsäureester der Formel (IIIa) 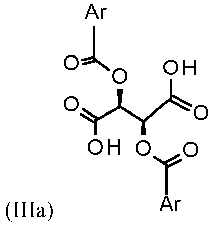 wobei Ar für einen unsubstituierten oder substituierten Aromaten oder Heteroaromaten steht.

3. Verfahren nach Anspruch 1 oder 2 **dadurch gekennzeichnet, dass** die Racematspaltung in Ethanol/ Wasser Gemisch durchgeführt wird.

4. Verfahren nach einem der Ansprüche 1 bis 3, **dadurch gekennzeichnet, dass** die Racematspaltung bei einer Temperatur im Bereich von 60 bis 80°C erfolgt.

5. Verfahren nach einem der Ansprüche 1 bis 4, **dadurch gekennzeichnet, dass** Ar für Phenyl steht.

6. Verfahren nach einem der Ansprüche 1 bis 5, **dadurch gekennzeichnet, dass** Dibenzoylweinsäure (V) 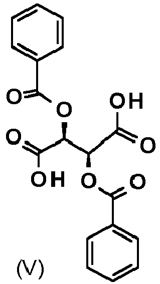 zur Racemattrennung eingesetzt wird.

7. Verfahren nach einem der Ansprüche 1 bis 6 **dadurch gekennzeichnet, dass** man das ausgefallene Diastereomerensalz (IVa), (IVb), (IVc) und oder (IVd) isoliert.

8. Verfahren nach einem der Ansprüche 1 bis 7, dadurch gekenzeichnet, dass man das Diastereomerensalzes mit einer Base behandelt und das Lösungsmittel entfernt.

9. Verfahren nach einem der Ansprüche 1 bis 8, **dadurch gekennzeichnet, dass** Kaliumhydroxid, Kaliumphosphat oder Natriumphosphat als Base eingesetzt werden.

10. Verfahren nach einem der Ansprüche 1 bis 9 wobei das Racemat 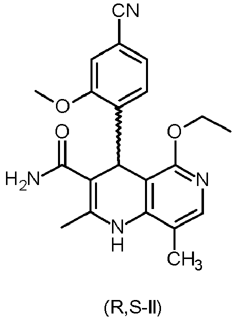 mit Dibenzoylweinsäure der Formel (V) 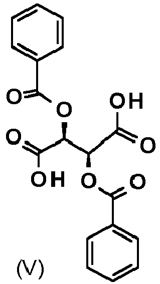 in einem Branntwein/ Wasser Gemisch zu dem Diastereomerensalz (VI) 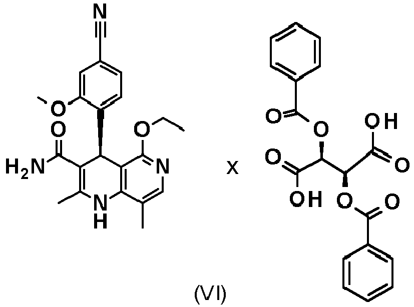 umgesetzt wird, und anschließend Finerenone (I) 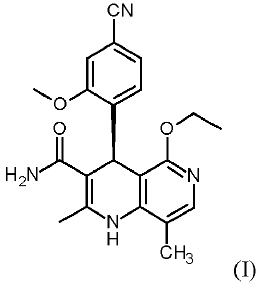 unter Einsatz von Natriumphosphat ebenfalls in einem Branntwein/ Wasser Gemisch freigesetzt wird.

11. Diastereomerensalze gemäß der Formel 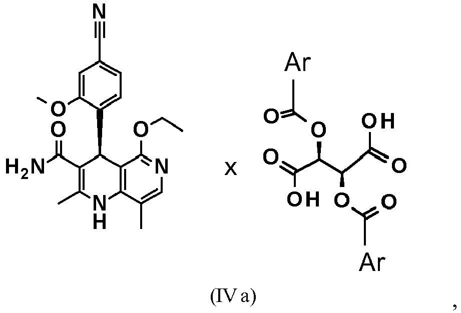 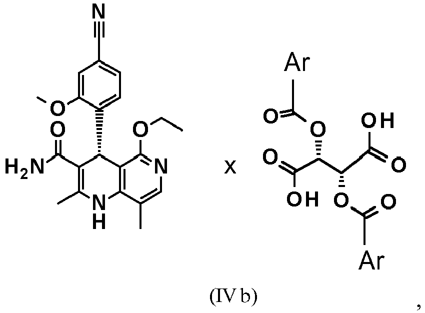 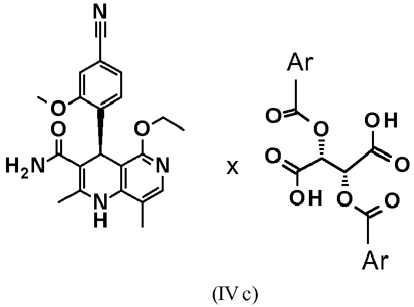 oder 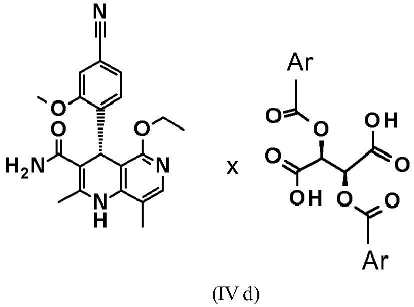 worin Ar für einen unsubstituierten oder substituierten Aromaten oder Heteroaromaten steht.

12. Diastereomerensalz gemäß Anspruch 11, **dadurch gekennzeichnet, dass** Ar für 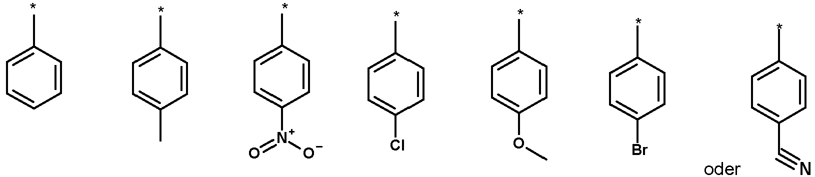 steht, worin * für die Verknüpfungsstelle steht.

13. Diastereomerensalz gemäß Anspruch 11 oder 12, **dadurch gekennzeichnet, dass** Ar für Phenyl steht.

14. Finerenone (I) mit einem Dibenzoylweinsäuregehalt ≤ 0,15% dadurch erhalten, dass man das Racemat (II) 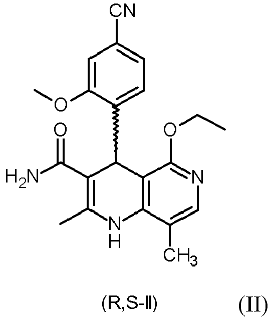 mit Dibenzoylweinsäure der Formel (V) 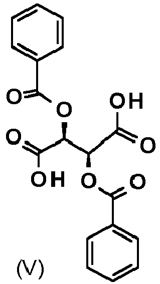 in einem Branntwein/ Wasser Gemisch zu dem Diastereomerensalz (VI) 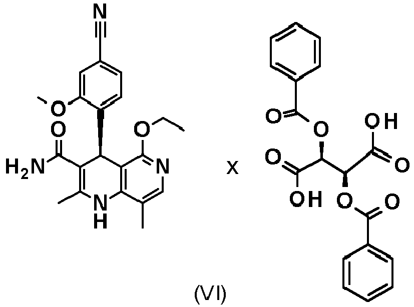 umsetzt und anschließend Finerenone (I) 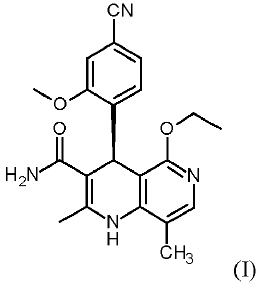 unter Einsatz von Natriumphosphat ebenfalls in einem Branntwein/ Wasser Gemisch freisetzt und anschließend erneut mit Natriumphosphat in Branntwein/ Wassergemisch umsetzt und dann reines Finerenone mit einem Dibenzoylweinsäuregehalt ≤ 0,15% auskristallisiert.

15. Finerenone (I) mit einem Dibenzoylweinsäuregehalt ≤ 0,1% erhalten nach einem Verfahren gemäß Anspruch 14.
